# EUROPEAN PATENT SPECIFICATION

(11) **EP 2 212 439 B1**
(45) Date of publication and mention of the grant of the patent: **25.03.2015**
(21) Application number: 08850164.8
(22) Date of filing: 11.11.2008
(51) Int. Cl.: C12Q 1/68

(54) **ZFP69,ZNF642 AND ZNF643 AS MARKERS FOR OBESITY-ASSOCIATED DIABETES**
ZFP69,ZNF642 UND ZNF643 ALS MARKER FÜR MIT OBESITÄT ASSOZIIERTEM DIABETES
ZFP69, ZNF642 ET ZNF643 EN TANT QUE MARQUEURS DES DIABÈTES LIÉS À L'OBÉSITÉ

(30) Priority: 12.11.2007 EP 07021922
(43) Date of publication of application: 04.08.2010
(73) Proprietor: Deutsches Institut für Ernährungsforschung, 14558 Nuthetal (DE)
(72) Inventor: JOOST, Hans-Georg, 14532 Kleinmachnow (DE); KLUGE, Reinhart, 14558 Nuthetal (DE); NESTLER, Matthias, 14471 Potsdam (DE); SCHERNECK, Stephan, 10407 Berlin (DE); SCHÜRMANN-BARTSCH, Anette, 14558 Nuthetal (DE)
(74) Representative: Krauss, Jan
(86) International application number: PCT/IB2008/003786
(87) International publication number: WO 2009/063324

(56) References cited:
- WO-A-2007/047796
- US-A1- 2005 266 423
- US-B1- 6 635 742
- DATABASE GENBANK [Online] NCBI; Version: NM_001005788.2 GI:118131096 4 July 2007 (2007-07-04), "Mus musculus zinc finger protein 69 (Zfp69), mRNA" XP002505478 Database accession no. NM_001005788
- SCHEEN A J: "Treatment of diabetes in patients with severe obesity" BIOMEDICINE AND PHARMACOTHERAPY, vol. 54, no. 2, March 2000 (2000-03), pages 74-79, XP009109210 ISSN: 0753-3322
- LEE D K H ET AL: "Chapter 24. Development of a gene expression-based screen using quantitative polymerase chain reaction" 1 January 1997 (1997-01-01), HIGH THROUGHPUT SCREENING : THE DISCOVERY OF BIOACTIVE SUBSTANCES, DEKKER, NEW YORK, PAGE(S) 413 - 426 , XP009108957 ISBN: 978-0-8247-0067-6 pages 413-414 the whole document
- PLUM LEONA ET AL: "Type 2 diabetes-like hyperglycemia in a backcross model of NZO and SJL mice: Characterization of a susceptibility locus on chromosome 4 and its relation with obesity" DIABETES, vol. 49, no. 9, September 2000 (2000-09), pages 1590-1596, XP002505474 ISSN: 0012-1797 cited in the application
- PLUM L ET AL: "Characterisation of the mouse diabetes susceptibility locus Nidd/SJL: Islet cell destruction, interaction with the obesity QTL Nob1, and effect of dietary fat" DIABETOLOGIA, vol. 45, no. 6, June 2002 (2002-06), pages 823-830, XP002505475 ISSN: 0012-186X cited in the application
- THOMPSON D B ET AL: "EVIDENCE FOR LINKAGE BETWEEN A REGION ON CHROMOSOME IP AND THE ACUTE INSULIN RESPONSE IN PIMA INDIANS" DIABETES, AMERICAN DIABETES ASSOCIATION, US, vol. 44, no. 4, 1 April 1995 (1995-04-01), pages 478-481, XP000929742 ISSN: 0012-1797 cited in the application
- PERRY JOHN R B ET AL: "New gene variants alter type 2 diabetes risk predominantly through reduced beta-cell function." CURRENT OPINION IN CLINICAL NUTRITION AND METABOLIC CARE JUL 2008, vol. 11, no. 4, July 2008 (2008-07), pages 371-377, XP009109151 ISSN: 1363-1950
- HUNTLEY STUART ET AL: "A comprehensive catalog of human KRAB-associated zinc finger genes: Insights into the evolutionary history of a large family of transcriptional repressors" GENOME RESEARCH, vol. 16, no. 5, May 2006 (2006-05), pages 669-677, XP002505477 ISSN: 1088-9051

### SEQUENCE LISTING

<110> DIfE - Deutsches Institut für Ernährungsforschung
<120> Zfp69, Znf642 and Znf643 as markers for obesity-associated diabetes
<130> D60226PCT
<150> EP 07021922.5
   <151> 12.11.2007
<160> 6
<170> PatentIn version 3.4
<210> 1
   <211> 1964
   <212> DNA
   <213> mus musculus
<400> 1
   tgatcacccc ggccactgag gcgacctggg tgaagttgaa ggaggtgtcc ctgtgggagg     60
   atgtgacaaa aatgtttgga ggagaagctc tgctatccca tgatgctaat gggacacaac    120
   aagaaagctt agctgatgga acgacccctg gcacccctgc agcaggatcc catgatggag    180
   cgacccctgg caccactgca acaggatccc atgatgaagc gacccctggc acccctgcag    240
   caggatccca tgatggagag acccctggca tccctgcagc aggatcccat gatggagaga    300
   cccctggcac ccctacagca ggatcccatg atggagtgac ccctggcacc actgcagcag    360
   gatcccagga atcgttgact ttcaaggaca ttgccgtgga cttatcccag gaggagtggg    420
   gacagctggc tcctgcatac caggatttgt atcgggaggt gatgctggag aactacagaa    480
   acctggtgtc agtggcagga tatcaacttt ccaagcctac tgtgatttcc cagttggaaa    540
   aaggagaggg accatgcatg gcagagagtc aaggcccaga ggatcccatt ttagatgtga    600
   agaataaact agaaaccaag gagtcaactg cagaggatga catttctgtc aagctcgatc    660
   atggcatcac gaggggaagg ctgatagagg atgatatagt ttgttctcca ttgaaaaaag    720
   cctccagcta cagtgataca ttagagagtc accgggcaac ctgtgggaaa ggcacgagac    780
   gagccatttg gacccacaaa aagaaacgcc aagaagggaa caaattggaa aacccagaga    840
   gttccaatgt tattttagaa cagaagcacc gtaaacacaa accagccaga aagaggaaca    900
   agtataaatt agattcaatt gatcacccag tgagctgtat gagagccaga aggtacccat    960
   gtaatgtgtg tgagaaaatg tttaagcaac ccattcacct tgtcgagcac atgagaactc   1020
   acactggcga gaaaccgttc cggtgcaagg agtgtgggag ggcctttagt cagagcgcat 1080
   ctctcaatac gcaccagagg atccacactg gggagaagcc cttcgcatgt gaggagtgcg 1140
   gcaaagcctt ccggcatcgc tcgtcgctta accagcatca ccggactcac actggagaga 1200
   aaccgttcac gtgtgacaaa tgtcagaaag cctttagcca gaacatcagc ttggtccagc 1260
   atctgaggac tcattcggga gagaaaccct ttagctgcag tgaatgtggg aaaccctttc 1320
   gacagattag acaccttagc gaacatgtaa gaatccacac tggggagaag ccctacaaat 1380
   gcacctcctg ctgtaaaaca ttcagtcata gagcatatct cacacatcac cagcgaatcc 1440
   acaccgggga gagaccctac aaatgtaagg agtgcgggaa agccttcagg cagaggatac 1500
   accttagcaa ccacaggact gttcacacag gggtgaaagc ctacgagtgt aaccgctgtg 1560
   ggaaagccta caggcacgac tcctccttta agaaacacca gcgacatcac acgggagaaa 1620
   agccttacga atgtactgag tgtggaaagt ccttcagcta taactcatcc ctgagccgac 1680
   accagaaaat ccacaggagg aacactttcc gagatgatcc gggacatgag aacaaaaggc 1740
   agctctaaat tggtgattct gagctttaaa gtttcaggac tcgagtaacc ttgagaaatt 1800
   aacaacaacg gtggcaactt ttgattttgc caaaagtgta aataaatacc ctgttgatta 1860
   ctactgtctg acaattacac aaaatgataa attaagcatt atgagaattt cacaaatatt 1920
   tttttactca tttcatgata gatgaataaa ttatttctta tgaa 1964
<210> 2
   <211> 2176
   <212> DNA
   <213> homo sapiens
<400> 2
   gatgggaatg tggagcagac cctcgtccac tccggaggcc gagggtcctc cgggcttccg 60
   aaggaagccg acctcaacgc tggacgcttc ttggagaatg attgcgttga gtggagatgt 120
   ggtctgtcta taaaaggccg ggagggaaca atatctgtta ccactcagtc cgtctctaaa 180
   gagacactct ttaccgctga aaacctcaag agtgagcact cccacgcccc cgtctctggt 240
   cctacctggg tccaaggccg atgtgaagtg gacaagtcca gtaaggcagg catcatgcca 300
   cagcagctcc tgatcaccct gcctaccgag gccagcacct gggtgaagct gcaacatcca 360
   aagaaggccg tggagggggc gcccctgtgg gaggatgtga ctaaaatgtt tgaaggagaa 420
   gctctgctgt ctcaggatgc tgaggacgta aagacccaga gagaaagttt agaggatgaa 480
   gtgacccctg gactcccgac agcagaatcc caggaattgt tgactttcaa ggacatatct 540
   attgacttca cccaggaaga gtgggggcag ctggctcctg ctcaccagaa tctataccga 600
   gaggtgatgc tggagaacta cagcaacttg gtgtcagtgg gatatcaact ttccaaacct 660
   agtgtgatat cccagttaga gaaaggagaa gagccatgga tggcagagaa agaaggccca 720
   ggagatccca gttcagactt gaagagtaaa atagaaacca ttgagtcaac tgcaaagagt 780
   accatttcac aggagcgctt atatcatggc attatgatgg aaagtttcat gagggatgat 840
   ataatttatt ccacgttgag aaaagtctcc acatatgatg atgtcttaga aaggcaccag 900
   gaaacttgta tgagagatgt gagacaagcc atcttgaccc ataagaagag agtccaagaa 960
   actaacaaat ttggggaaaa tatcattgtg cattcaaatg ttattattga acagaggcac 1020
   cataaatatg atacacctac aaagcggaac acatacaaat tagatttgat taatcatcca 1080
   acaagttaca taagaacaaa aacctatgaa tgtaatatat gtgaaaaaat cttcaaacaa 1140
   cctattcacc ttactgaaca tatgagaatt catactggtg agaaaccttt cagatgtaag 1200
   gaatgtggaa gggcctttag tcaaagtgca tccctcagta cacaccagag aatccatact 1260
   ggtgagaaac cctttgaatg tgaggaatgt gggaaagcct tcagacatcg ctcatcactt 1320
   aatcagcatc atagaactca cactggggag aaaccctatg tatgtgataa atgtcagaaa 1380
   gctttcagcc agaacattag cttggttcaa catttgagga ctcattctgg agagaaacct 1440
   tttacttgca atgaatgtgg gaaaaccttt agacagatta gacaccttag tgaacatata 1500
   agaattcata ccggggagaa gccctatgca tgcactgcat gttgtaaaac ctttagtcat 1560
   agagcgtatc taacacatca ccagagaatc catactgggg agagacccta caaatgtaaa 1620
   gaatgtggaa aagcctttag gcagaggata caccttagca accataaaac tgttcataca 1680
   ggagtgaaag catatgaatg caaccgctgt ggaaaagcct ataggcatga ttcatccttt 1740
   aaaaaacatc agagacatca cactggagaa aaaccttacg aatgtaacga atgtggaaaa 1800
   gccttcagct ataactcttc acttagtcga catcatgaaa tacacaggag gaacgccttc 1860
   cgaaataagg tgtaaaaaca gatatttgac ttgagaacaa aagccaagtg taaattggtg 1920
   atttagagtg ctttaaaatt tcaggactca gatatgagga attgatgtaa tgatgccaac 1980
   ttttaatttt tcccattgta aataaacatt acattgacag gtattgacta ctaacacctc 2040
   taaaaagtac ttaagattaa aatctggtcc ttaaaattaa atgaattcag cattatgaaa 2100
   aattcataac agtttttttc tgatttcatg gtggattaat aaattattct tcatgggtaa 2160
   aaaaaaaaaa aaaaaa 2176
<210> 3
   <211> 1976
   <212> DNA
   <213> homo sapiens
<400> 3
   ggccgcgcag tgccggaacc cggctgcagc ggtgggaagg cgggggcgtg ccggcccagc 60
   ggggagaggg ggctgctgag agtaaatact tggcgcctcc agctgctggc caaggagaca 120
   gatggagctc aagttgggag atacgccctg agagccgatg atagacacaa gtccagatct 180
   cggattttga tactgtatgt tccctgggtt cctgagagag gacattgagg agtaggagtc 240
   ggcgattaag gagatcggta caattgggaa gcctcctgtc agagcttcca gcaatttcct 300
   catcagaggt ggacaagccc tatgggctaa gacagagggt cctcagaaag gagtgcggac 360
   gccgtcatgc tgcagcagct cctgatcacc ctgcccaccg aggccagcac ctgggtgaag 420
   ttgcgtcatc caaaggcggc cacggagcgg gtggccctgt gggaggatgt gactaagatg 480
   tttaaagcag aaggaactgt taaccttcaa ggacgtatct gtggacttca ctcaggagga 540
   gtgggggcag ctggcccctg ctcaccggaa tctgtaccgg gaggtgatgc tggagaacta 600
   tgggaacctg gtctcagtgg gatgtcagct ttccaaacct ggcgtgattt cccagttgga 660
   gaaaggagaa gaaccatggc tgatggagag agatatttca ggagttccaa gttcagactt 720
   gaagagcaaa acaaaaacca aagagtcagc cttacagaat gatatttcgt gggaagaact 780
   acattgtggc ctaatgatgg aaagatttac aaaaggaagc agcatgtatt ccaccttggg 840
   aagaatctcc aaatgtaata agctagaaag ccaacaagag aaccaaagaa tgggtaaggg 900
   gcaaatcccc ctgatgtgca agaaaacatt cactcaggag agaggccaag agtctaatag 960
   atttgagaaa agaattaatg tgaagtcaga agttatgcca ggaccaatag gtcttccaag 1020
   aaaaagagat cgtaaatatg acacacctgg aaagagaagc agatacaaca tagatttagt 1080
   taatcattca aggagttata caaaaatgaa aacctttgag tgtaatattt gtgaaaaaat 1140
   cttcaaacag cttattcacc ttactgaaca catgagaatt cataccgggg agaaaccttt 1200
   cagatgtaag gaatgtggaa aagcctttag ccaaagttca tctcttattc cgcatcagag 1260
   aattcatact ggtgagaaac cctatgaatg taaggagtgt gggaaaacct tcagacatcc 1320
   ttcatcgctt actcaacatg ttagaattca taccggggaa aagccctatg aatgtagggt 1380
   atgtgagaaa gccttcagcc agagcattgg actgatccag catttgagaa ctcatgttag 1440
   agagaaacct tttacatgca aagactgtgg aaaagcgttt ttccagatta gacaccttag   1500
   gcaacatgag attattcata ctggtgtgaa accctatatt tgtaatgtat gtagtaaaac   1560
   cttcagccat agtacatacc taactcaaca ccagagaact catactggag aaagaccata   1620
   taaatgtaag gaatgtggga aagcctttag ccagagaata catctttcta tccatcagag   1680
   agtccatact ggagtaaaac cttatgaatg cagtcattgt gggaaagcct ttaggcatga   1740
   ttcatccttt gctaaacatc agagaattca tactggagaa aaaccttatg attgtaatga   1800
   gtgtggaaaa gccttcagct gtagttcatc ccttattaga cactgcaaaa cacatttaag   1860
   aaataccttc agcaatgttg tgtgaaatat actaaacatc aaagaatcta tgttggagca   1920
   caagattcta aatcagtggt tccctgatcc ctcaaaaatc catttgtttt tggatt       1976
<210> 4
   <211> 427
   <212> PRT
   <213> mus musculus
<400> 4
   Met Leu Glu Asn Tyr Arg Asn Leu Val Ser Val Ala Gly Tyr Gln Leu
   1               5                   10                  15
   Ser Lys Pro Thr Val Ile Ser Gln Leu Glu Lys Gly Glu Gly Pro Cys
               20                  25                  30
   Met Ala Glu Ser Gln Gly Pro Glu Asp Pro Ile Leu Asp Val Lys Asn
           35                  40                  45
   Lys Leu Glu Thr Lys Glu Ser Thr Ala Glu Asp Asp Ile Ser Val Lys
       50                  55                  60
   Leu Asp His Gly Ile Thr Arg Gly Arg Leu Ile Glu Asp Asp Ile Val
   65                  70                  75                  80
   Cys Ser Pro Leu Lys Lys Ala Ser Ser Tyr Ser Asp Thr Leu Glu Ser
                   85                  90                  95
   His Arg Ala Thr Cys Gly Gly Thr Arg Arg Ala Ile Trp Thr His Lys
               100                 105                 110
   Lys Lys Arg Gln Glu Gly Asn Lys Leu Glu Asn Pro Glu Ser Ser Asn
           115                 120                 125
   Val Ile Leu Glu Gln Lys His Arg Lys His Lys Pro Ala Arg Lys Arg
       130                 135                     140
   Asn Lys Tyr Lys Leu Asp Ser Ile Asp His Pro Val Ser Cys Met Arg
   145                 150                 155                     160
   Ala Arg Arg Tyr Pro Cys Asn Val Cys Glu Lys Met Phe Lys Gln Pro
                   165                 170                     175
   Ile His Leu Val Glu His Met Arg Thr His Thr Gly Glu Lys Pro Phe
               180                 185                     190
   Arg Cys Lys Glu Cys Gly Arg Ala Phe Ser Gln Ser Ala Ser Leu Asn
           195                 200                     205
   Thr His Gln Arg Ile His Thr Gly Glu Lys Pro Phe Ala Cys Glu Glu
       210                 215                     220
   Cys Gly Lys Ala Phe Arg His Arg Ser Ser Leu Asn Gln His His Arg
   225                 230                 235                     240
   Thr His Thr Gly Glu Lys Pro Phe Thr Cys Asp Lys Cys Gln Lys Ala
                   245                 250                     255
   Phe Ser Gln Asn Ile Ser Leu Val Gln His Leu Arg Thr His Ser Gly
               260                 265                     270
   Glu Lys Pro Phe Ser Cys Ser Glu Cys Gly Lys Pro Phe Arg Gln Ile
           275                 280                     285
   Arg His Leu Ser Glu His Val Arg Ile His Thr Gly Glu Lys Pro Tyr
       290                 295                     300
   Lys Cys Thr Ser Cys Cys Lys Thr Phe Ser His Arg Ala Tyr Leu Thr
   305                 310                 315                     320
   His His Gln Arg Ile His Thr Gly Glu Arg Pro Tyr Lys Cys Lys Glu
                   325                 330                     335
   Cys Gly Lys Ala Phe Arg Gln Arg Ile His Leu Ser Asn His Arg Thr
               340                 345                     350
   Val His Thr Gly Val Lys Ala Tyr Glu Cys Asn Arg Cys Gly Lys Ala
   355 360 365
   Tyr Arg His Asp Ser Ser Phe Lys Lys His Gln Arg His His Thr Gly
   370 375 380
   Glu Lys Pro Tyr Glu Cys Thr Glu Cys Gly Lys Ser Phe Ser Tyr Asn
   385 390 395 400
   Ser Ser Leu Ser Arg His Gln Lys Ile His Arg Arg Asn Thr Phe Arg
   405 410 415
   Asp Asp Pro Gly His Glu Asn Lys Arg Gln Leu
   420 425
<210> 5
   <211> 526
   <212> PRT
   <213> homo sapiens
<400> 5
   Met Pro Gln Gln Leu Leu Ile Thr Leu Pro Thr Glu Ala Ser Thr Trp
   1 5 10 15
   Val Lys Leu Gln His Pro Lys Lys Ala Val Glu Gly Ala Pro Leu Trp
   20 25 30
   Glu Asp Val Thr Lys Met Phe Glu Gly Glu Ala Leu Leu Ser Gln Asp
   35 40 45
   Ala Glu Asp Val Lys Thr Gln Arg Glu Ser Leu Glu Asp Glu Val Thr
   50 55 60
   Pro Gly Leu Pro Thr Ala Glu Ser Gln Glu Leu Leu Thr Phe Lys Asp
   65 70 75 80
   Ile Ser Ile Asp Phe Thr Gln Glu Glu Trp Gly Gln Leu Ala Pro Ala
   85 90 95
   His Gln Asn Leu Tyr Arg Glu Val Met Leu Glu Asn Tyr Ser Asn Leu
   100 105 110
   Val Ser Val Gly Tyr Gln Leu Ser Lys Pro Ser Val Ile Ser Gln Leu
   115 120 125
   Glu Lys Gly Glu Glu Pro Trp Met Ala Glu Lys Glu Gly Pro Gly Asp
   130 135 140
   Pro Ser Ser Asp Leu Lys Ser Lys Ile Glu Thr Ile Glu Ser Thr Ala
   145 150 155 160
   Lys Ser Thr Ile Ser Gln Glu Arg Leu Tyr His Gly Ile Met Met Glu
   165 170 175
   Ser Phe Met Arg Asp Asp Ile Ile Tyr Ser Thr Leu Arg Lys Val Ser
   180 185 190
   Thr Tyr Asp Asp Val Leu Glu Arg His Gln Glu Thr Cys Met Arg Asp
   195 200 205
   Val Arg Gln Ala Ile Leu Thr His Lys Lys Arg Val Gln Glu Thr Asn
   210 215 220
   Lys Phe Gly Glu Asn Ile Ile Val His Ser Asn Val Ile Ile Glu Gln
   225 230 235 240
   Arg His His Lys Tyr Asp Thr Pro Thr Lys Arg Asn Thr Tyr Lys Leu
   245 250 255
   Asp Leu Ile Asn His Pro Thr Ser Tyr Ile Arg Thr Lys Thr Tyr Glu
   260 265 270
   Cys Asn Ile Cys Glu Lys Ile Phe Lys Gln Pro Ile His Leu Thr Glu
   275 280 285
   His Met Arg Ile His Thr Gly Glu Lys Pro Phe Arg Cys Lys Glu Cys
   290 295 300
   Gly Arg Ala Phe Ser Gln Ser Ala Ser Leu Ser Thr His Gln Arg Ile
   305 310 315 320
   His Thr Gly Glu Lys Pro Phe Glu Cys Glu Glu Cys Gly Lys Ala Phe
   325 330 335
   Arg His Arg Ser Ser Leu Asn Gln His His Arg Thr His Thr Gly Glu
   340 345 350
   Lys Pro Tyr Val Cys Asp Lys Cys Gln Lys Ala Phe Ser Gln Asn Ile
   355 360 365
   Ser Leu Val Gln His Leu Arg Thr His Ser Gly Glu Lys Pro Phe Thr
   370 375 380
   Cys Asn Glu Cys Gly Lys Thr Phe Arg Gln Ile Arg His Leu Ser Glu
   385 390 395 400
   His Ile Arg Ile His Thr Gly Glu Lys Pro Tyr Ala Cys Thr Ala Cys
   405 410 415
   Cys Lys Thr Phe Ser His Arg Ala Tyr Leu Thr His His Gln Arg Ile
   420 425 430
   His Thr Gly Glu Arg Pro Tyr Lys Cys Lys Glu Cys Gly Lys Ala Phe
   435 440 445
   Arg Gln Arg Ile His Leu Ser Asn His Lys Thr Val His Thr Gly Val
   450 455 460
   Lys Ala Tyr Glu Cys Asn Arg Cys Gly Lys Ala Tyr Arg His Asp Ser
   465 470 475 480
   Ser Phe Lys Lys His Gln Arg His His Thr Gly Glu Lys Pro Tyr Glu
   485 490 495
   Cys Asn Glu Cys Gly Lys Ala Phe Ser Tyr Asn Ser Ser Leu Ser Arg
   500 505 510
   His His Glu Ile His Arg Arg Asn Ala Phe Arg Asn Lys Val
   515 520 525
<210> 6
   <211> 432
   <212> PRT
   <213> homo sapiens
<400> 6
   Met Leu Glu Asn Tyr Gly Asn Leu Val Ser Val Gly Cys Gln Leu Ser
   1               5                   10                  15
   Lys Pro Gly Val Ile Ser Gln Leu Glu Lys Gly Glu Glu Pro Trp Leu
               20                  25                  30
   Met Glu Arg Asp Ile Ser Gly Val Pro Ser Ser Asp Leu Lys Ser Lys
           35                  40                  45
   Thr Lys Thr Lys Glu Ser Ala Leu Gln Asn Asp Ile Ser Trp Glu Glu
       50                  55                  60
   Leu His Cys Gly Leu Met Met Glu Arg Phe Thr Lys Gly Ser Ser Met
   65                  70                  75                  80
   Tyr Ser Thr Leu Gly Arg Ile Ser Lys Cys Asn Lys Leu Glu Ser Gln
                   85                  90                  95
   Gln Glu Asn Gln Arg Met Gly Lys Gly Gln Ile Pro Leu Met Cys Lys
               100                 105                 110
   Lys Thr Phe Thr Gln Glu Arg Gly Gln Glu Ser Asn Arg Phe Glu Lys
           115                 120                 125
   Arg Ile Asn Val Lys Ser Glu Val Met Pro Gly Pro Ile Gly Leu Pro
       130                 135                 140
   Arg Lys Arg Asp Arg Lys Tyr Asp Thr Pro Gly Lys Arg Ser Arg Tyr
   145                 150                 155                 160
   Asn Ile Asp Leu Val Asn His Ser Arg Ser Tyr Thr Lys Met Lys Thr
                   165                 170                 175
   Phe Glu Cys Asn Ile Cys Glu Lys Ile Phe Lys Gln Leu Ile His Leu
               180                 185                 190
   Thr Glu His Met Arg Ile His Thr Gly Glu Lys Pro Phe Arg Cys Lys
           195                 200                 205
   Glu Cys Gly Lys Ala Phe Ser Gln Ser Ser Ser Leu Ile Pro His Gln
       210                 215                 220
   Arg Ile His Thr Gly Glu Lys Pro Tyr Glu Cys Lys Glu Cys Gly Lys
   225 230 235 240
   Thr Phe Arg His Pro Ser Ser Leu Thr Gln His Val Arg Ile His Thr
   245 250 255
   Gly Glu Lys Pro Tyr Glu Cys Arg Val Cys Glu Lys Ala Phe Ser Gln
   260 265 270
   Ser Ile Gly Leu Ile Gln His Leu Arg Thr His Val Arg Glu Lys Pro
   275 280 285
   Phe Thr Cys Lys Asp Cys Gly Lys Ala Phe Phe Gln Ile Arg His Leu
   290 295 300
   Arg Gln His Glu Ile Ile His Thr Gly Val Lys Pro Tyr Ile Cys Asn
   305 310 315 320
   Val Cys Ser Lys Thr Phe Ser His Ser Thr Tyr Leu Thr Gln His Gln
   325 330 335
   Arg Thr His Thr Gly Glu Arg Pro Tyr Lys Cys Lys Glu Cys Gly Lys
   340 345 350
   Ala Phe Ser Gln Arg Ile His Leu Ser Ile His Gln Arg Val His Thr
   355 360 365
   Gly Val Lys Pro Tyr Glu Cys Ser His Cys Gly Lys Ala Phe Arg His
   370 375 380
   Asp Ser Ser Phe Ala Lys His Gln Arg Ile His Thr Gly Glu Lys Pro
   385 390 395 400
   Tyr Asp Cys Asn Glu Cys Gly Lys Ala Phe Ser Cys Ser Ser Ser Leu
   405 410 415
   Ile Arg His Cys Lys Thr His Leu Arg Asn Thr Phe Ser Asn Val Val
   420 425 430

## Description

The present invention relates to the genes *Zfp69*, *ZNF642* or *ZNF643* and their use as genetic markers in obesity-related diabetes. *Zfp69*, *ZNF642* or *ZNF643* provide valuable tools both for diagnostic as well as therapeutic approaches, in order to treat or prevent obesity-related diabetes.

### Description

Type 2 diabetes results from the combination of insulin resistance and inadequate insulin secretion, the former being associated with obesity [Kahn, 1998]. The risk of developing type 2 diabetes is to approximately 50% inherited [Florez et al., 2003]. Recently, numerous associations between single nucleotide polymorphisms and the diabetes risk in humans have been identified and confirmed [Sladek et al., 2007, Scott et al., 2007, Steinthorsdottir et al., 2007, Saxena et al., 2007, Zeggini et al., 2007]. However, the functional consequences of these SNPs at the molecular, cellular and physiological level are almost entirely unknown.

Obese mouse strains carrying the ob/ob or the db/db mutation have proven to be valuable models for the study of the pathophysiology and genetics of type 2 diabetes [Herberg and Coleman, 1977]. In these strains, the adipogenic mutation is necessary, but not sufficient for the development of severe hyperglycaemia and diabetes [Herberg and Leiter, 2001]. Thus, the diabetic phenotype appeared to be conferred by the background strain, and it was assumed that lean mice may carry diabetogenic and/or diabetes-protecting alleles. Furthermore, with outcross experiments separating quantitative trait loci for obesity and hyperglycaemia, the concept was proven that diabetes is the result of a combination of adipogenic and diabetogenic alleles [Leiter et al., 1998; Plum et al., 2000; Plum et al., 2002; Reifsnyder and Leiter, 2002]. In these experiments, diabetogenic alleles from the lean strains NON or SJL were introduced into the New Zealand Obese (NZO) strain, conferring severe hyperglycaemia and beta cell failure in the outcross population. More recently, a gene involved in microvasculature function (Sorcs1) has been identified as a contributor to diabetes in BTBR-ob/ob mice [Clee et al., 2006]. Thus, positional cloning may provide major insights into the pathogenesis of obesity-associated diabetes.

Obesity is a complex genetic disease. Although it is clearly heritable (Price, R.A., Ness, R. and Laskarzewski, P. (1990) Common major gene inheritance of extreme overweight. Hum. Biol., 62, 747-765. Hunt, S.C., Hasstedt, S.J., Kuida, H., Stults, B.M., Hopkins, P.H. and Williams, R.R. (1989) Genetic heritability and common environmental components of resting and stressed blood pressures, lipids, and body mass index in Utah pedigrees and twins. Am. J. Epidemiol., 129, 625-638. Bell, C.G., Walley, A.J. and Froguel, P. (2005) The genetics of human obesity. Nat. Rev. Genet., 6, 221-234.) (e.g. first-degree relatives of obese patients have up to a 9-fold increase in disease risk (Price, R.A. and Lee, J.H. (2001) Risk ratios for obesity in families of obese African-American and Caucasian women. Hum. Hered., 51, 35-40.), the segregation of the disease in obesity-prone families is non-Mendelian (Borecki, I.B., Higgins, M., Schreiner, P.J., Arnett, D.K., Mayer-Davis, E., Hunt, S.C. and Province, M.A. (1998) Evidence for multiple determinants of the body mass index: the National Heart, Lung, and Blood Institute Family Heart Study. Obes. Res., 6, 107-114.). Also, many studies have provided evidence to suggest that risk for obesity is determined not only by specific genotypes but also by significant gene/gene and gene/environment interactions (Dong, C., Wang, S., Li, W.D., Li, D., Zhao, H. and Price, R.A. (2003) Interacting genetic loci on chromosomes 20 and 10 influence extreme human obesity. Am. J. Hum. Genet., 72, 115-124. Dong, C., Li, W.D., Li, D. and Price, R.A. (2005) Interaction between obesity-susceptibility loci in chromosome regions 2p25-p24 and 13q13-q21. Eur. J. Hum. Genet., 13, 102-108. Warden, C.H., Yi, N. and Fisler, J. (2004) Epistasis among genes is a universal phenomenon in obesity: evidence from rodent models. Nutrition, 20, 74-77. Lewis, C.E., North, K.E., Arnett, D., Borecki, I.B., Coon, H., Ellison, R.C., Hunt, S.C., Oberman, A., Rich, S.S. and Province, M.A.(2005) Sex-specific findings from a genome-wide linkage analysis of human fatness in non-Hispanic whites and African Americans: the HyperGEN study. Int. J. Obes. Relat. Metab. Disord., 29, 639-649).

There have been many attempts to identify genes involved in the predisposition to common obesity, but presumably because of the genetic complexity, these efforts have had only mixed success (Bell, C.G., Walley, A.J. and Froguel, P. (2005) The genetics of human obesity. Nat. Rev. Genet., 6, 221-234., Pérusse, L., Rankinen, T., Zuberi, A., Chagnon, Y.C., Weisnagel, S.J., Argyropoulos, G., Walts, B., Snyder, E.E. and Bouchard, C. (2005) The human obesity gene map: the 2004 update. Obes. Res., 13, 381-490). Studies focused on rare forms of obesity have been more productive. Mutations in several genes have been linked to monogenic obesity. Also, several genes have been implicated in syndromic obesity (i.e. obesity with other clinically significant phenotypes not usually associated with the disease). However, genetic causes of the common form of this disease remain poorly defined.

Dietary fat intake is considered a major factor in the development of obesity and insulin resistance. In mice, sensitivity to dietary fat is strain specific, and consequently dependent on a genetic basis (West, D.B., Boozer, C.N., Moody, D.L. and Atkinson, R.L. Dietary obesity in nine inbred mouse strains. Am J Physiol 262, R1025-32 (1992). Wuschke, S., Dahm, S., Schmidt, C., Joost, H.G. and Al-Hasani, H. A meta-analysis of quantitative trait loci associated with body weight and adiposity in mice. Int J Obes Lond) (2006) Rankinen, T. et al. The human obesity gene map: the 2005 update. Obesity (Silver Spring) 14, 529-644 (2006)). Weight gain in response to dietary fat/caloric intake is dependent on the genetic composition of an individual. However, the genes and variants that determine the sensitivity to dietary fat are largely unknown. Since obesity is a major risk factor for the development of type-2 diabetes mellitus, cardiovascular diseases, and perhaps certain types of cancer, it is imperative to identify genetic risk factors for diet-induced adiposity and weight gain. Moreover, it is well established that obesity coincides with reduced insulin sensitivity and promotes impaired glucose tolerance.

ZFP69 is a member of the subfamily of zinc finger transcription factors that comprise an N-terminal KRAB and a zinc finger binding C2-H2 domain (Fig. 5H). Database entry NM_001005788 discloses the mRNA and protein sequences of the mouse zinc finger protein 69 (ZFP69). Database entry NM_198494 discloses the mRNA and protein sequences of the human zinc finger protein 642 (ZNF642). Database entry NM_023070 discloses the mRNA and protein sequences of the less related human zinc finger protein 643 (ZNF643).

Halama et al (in: Halama N, Yard-Breedijk A, Vardarli I, Akkoyun I, Yard B, Janssen B, van der Woude FJ. The Kruppel-like zinc-finger gene ZNF236 is alternatively spliced and excluded as susceptibility gene for diabetic nephropathy. Genomics. 2003 Sep;82(3):406-11.) describe the construction of a transcript map of the target region on chromosome 18q22.3-q23 and analysis of the candidate gene ZNF236 in the context of diabetic nephropathy. ZNF236 was sequenced in patients with diabetic nephropathy and diabetes without nephropathy, as well as in unaffected controls. Multiple splice forms in all individuals were observed, but no mutation in any of the patients. It therefore seemed improbable that ZNF236 is a gene that confers DN susceptibility.

Bouchard et al (in Bouchard L, Vohl MC, Deshaies Y, Rhéaume C, Daris M, Tchemof A. Visceral adipose tissue zinc finger protein 36 mRNA levels are correlated with insulin, insulin resistance index, and adiponectinemia in women. Eur J Endocrinol. 2007 Oct;157(4):451-7.) describe the transcriptome of omental adipose tissue, leading to the identification of a new candidate gene for obesity-related metabolic complications, zinc finger protein 36 (ZFP36), which is known to down-regulate tumor necrosis factor-alpha TNF-alpha) expression. No correlation was observed between s.c. ZFP36 mRNA levels and any of the phenotypes tested, and it was speculated that ZFP36 gene expression in omental adipose tissue, but not in abdominal s.c. fat, may offer partial protection against the development of insulin resistance and diabetes.

Gutiérrez-Aguilar et al. (in Gutiérrez-Aguilar R, Benmezroua Y, Vaillant E, Balkau B, Marre M, Charpentier G, Sladek R, Froguel P, Neve B. Analysis of KLF transcription factor family gene variants in type 2 diabetes. BMC Med Genet. 2007 Aug 9;8:53.) describe that the Krüppel-like factor (KLF) family consists of transcription factors as implicated in glucose homeostasis, making them candidate genes for involvement in type 2 diabetes (T2D). Variants of nine KLF genes were genotyped in T2D cases and controls and analysed in a two-stage study. Three of 28 SNPs as analysed showed a trend to be associated with T2D in our first case-control set (P < 0.10), but analysis of this set and the combined analysis of the three variants in all 2,219 individuals did not show an association with T2D in this population.

US 2005-266423 discloses the use of sequences 3 and 6 of the present application for the treatment of disorders.

In view of the above, it is an object of the present invention, to provide novel genetic markers that are involved in obesity-related diabetes. These markers provide valuable tools both for diagnostic as well as therapeutic approaches in order to treat or prevent obesity-related diabetes.

According to a first aspect thereof, the object of the present invention is solved by providing a method for diagnosing obesity-related diabetes or an increased risk for obesity-related diabetes in a mammal, comprising analyzing the expression of the full-length gene for mammalian ZPF69, preferably for human ZNF642 and/or human ZNF643, in a biological sample obtained from a mammal to be diagnosed, wherein a lack or reduced expression of the full-length gene for ZFP69, ZNF642 and/or ZNF643 in said mammal, when compared to a biological sample obtained from a non-diseased mammal, is indicative for a normal or for a reduced risk for obesity-related diabetes in said mammal. In the context of the present invention, ZNF642 and ZNF643 are understood as indicating/representing the human homolog of the mouse gene Zfp69, preferred is ZNF642.

The shorter mRNA for *Zfp69* generated in B6 and New Zealand obese (NZO) mice encodes a truncated protein which can be considered a loss-of-function variant, since it lacks both the KRAB and the (DNA binding) C2H2 domain of the transcription factor. It can be assumed that similar loss-of-function variants can be found in the human homologs of ZFP69, namely ZNF642 and ZNF643.

According to another aspect thereof, the object of the present invention is solved by providing a polynucleotide according to SEQ ID NO: 4, 5 or 6, a polypeptide that is encoded by SEQ ID NO: 4, 5 or 6, in particular having an amino acid sequence of SEQ ID NO: 1, 2 or 3, a polypeptide having an amino acid sequence having a 90% or more homology with an amino acid sequence of SEQ ID NO: 1, 2 or 3, and exhibiting a ZFP69, ZNF642 or ZNF643 activity, or a polypeptide having an amino acid sequence in which 1 to 10 amino acids are deleted, substituted, and/or added in the amino acid sequence according to SEQ ID NO: 1, 2 or 3, and exhibiting a ZFP69, ZNF642 or ZNF643 activity for use in the treatment of diseases.

According to yet another aspect thereof, the object of the present invention is solved by providing a method of identifying a compound for treating or preventing obesity-related diabetes through modulating the expression of the full-length gene for ZFP69, preferably for human ZNF642 and/or human ZNF643, in a cell, comprising the steps of a) contacting a cell expressing ZFP69, ZNF642 and/or ZNF643 with at least one potentially interacting compound, and b) measuring the modulation of the expression of ZFP69, ZNF642 and/or ZNF643 in said cell. Preferred is a method according to the present invention, wherein said compound as identified is an inhibitor of the expression of ZFP69, ZNF642 or ZNF643 in said cell.

The inventors have previously identified a QTL (Nidd/SJL) on distal chromosome 4 that aggravated and accelerated diabetes in an outcross population of NZO with the lean SJL strain [Plum et al., 2000; Plum et al., 2002]. This QTL exhibited high LOD scores for the trait blood glucose, produced beta cell destruction, and reproducibly doubled the prevalence of diabetes in a NZO-SJL backcross population [Plum et al., 2002]. It mapped to a similar position as Nidd1 that was identified in an outcross of NZO with NON [Leiter et al., 1998]. Both Nidd/SJL and Nidd1 map to a syntenic region of human chromosome 1 which comprises a QTL for reduced insulin secretion that was identified in the Pima Indian population

[Thompson et al., 1995]. Thus, Nidd/SJL appeared to be a prime target for positional cloning of a diabetes QTL.

After characterization of the region as described in the examples below, the only differentially expressed gene in the region was *Zfp69.*

In view of this, possible applications of the present invention for humans include:
a) Diagnostic approaches: Since ZFP69, ZNF642 or ZNF643 activity determines the susceptibility for obesity-related diabetes, genetic tests can be developed to assess an individual risk in relation to the magnitude of obesity, and to develop a personalized diet and/or treatment as an intervention approach.
b) Pharmaceutical (obesity/diabetes) and therapeutic approaches: The inventors' data indicate that an inhibition of the ZFP69, ZNF642 or ZNF643 action can be used as drug preventing or slowing down the course of type 2 diabetes in mammals/humans. A *Zfp69* or *ZNF642* inhibitor/inhibiting construct can be used as a diabetes drug as well, and also for treating other components of the metabolic syndrome, including hyperlipidemia, hypercholesterinemia, high blood pressure, and aspects of vascular and cardiovascular diseases that are usually secondary to insulin resistance and diabetes.

Moreover, *Zfp69, ZNF642* or *ZNF643* appear to define a novel pathway with potentially valuable downstream targets that regulate lipid metabolism, energy homeostasis and/or other, yet unknown biological entities.

The term "homology" as used herein means a value obtained by a BLAST [Basic local alignment search tool; Altschul, S. F. et al., J. Mol. Biol., 215, 403-410, (1990)] search. The homo logy in the amino acid sequence may be calculated by a BLAST search algorithm. More particularly, it may be calculated using a b12seq program (Tatiana A. Tatusova and Thomas L. Madden, FEMS Microbiol. Lett., 174, 247-250, 1999) in a BLAST package (sgi32bit edition, version 2.0.12; obtained from NCBI) in accordance with a default parameter. As a pairwise alignment parameter, a program "blastp" is used. Further, "0" as a Gap insertion cost value, "0" as a Gap elongation cost value, "SEG" as a filter for a query sequence, and "BLOSUM62" as a matrix are used, respectively.

Preferred is a diagnostic method according to the present invention, wherein said mammal to be diagnosed is a mouse, rat or human.

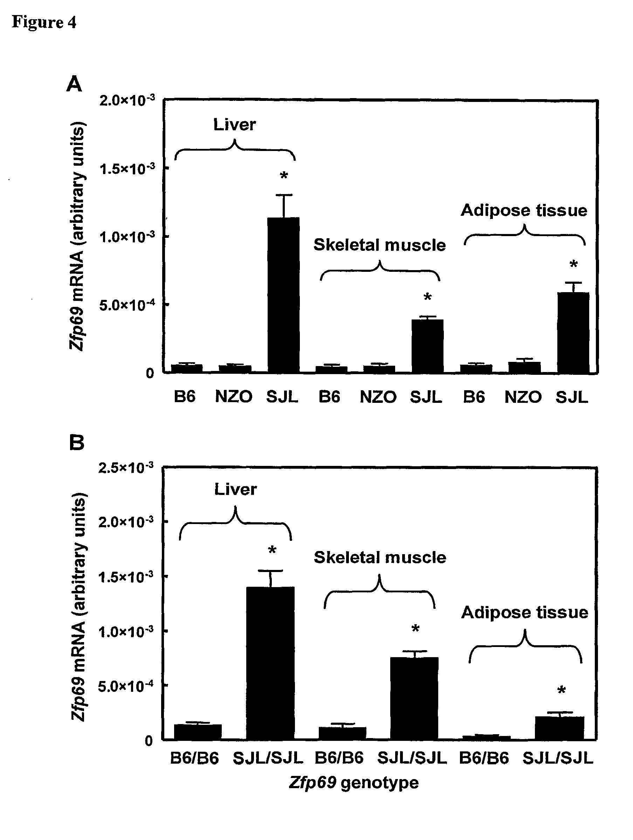

Preferred is a diagnostic method according to the present invention, wherein said biological sample contains biological material that is derived from liver, skeletal muscle, adipose tissue, heart, pancreas, kidney, and/or hypothalamus of said mammal to be diagnosed. In the mice as analysed, *Zfp69* is expressed mainly in liver, skeletal muscle, and adipose tissue (see Figure 4a).

Both the expression and/or abundance of ZFP69, ZNF642 or ZNF643 or the mRNAs thereof as well as the biological activities can be determined with any suitable assay known to the person of skill. Common assays involve PCR, Northern-, Western- or Southern-Blots, and/or enzymatic assays. Thus, preferred is a method according to the present invention, wherein said diagnosis comprises the use of ZFP69-, ZNF642- or ZNF643-specific antibodies, hybridisation analysis, quantitative PCR, mRNA analysis, quantitative analysis of the amount of ZFP69-, ZNF642- or ZNF643-protein, and/or sequencing of the *Zfp69-, ZNF642-* or *ZNF643*-locus in said sample.

Further preferred is a method according to the present invention, wherein said diagnoses comprises the analysis of the truncation of the mRNA of *Zfp69, ZNF642* and/or *ZNFf643* and/or analyzing SNPs in the chromosomal *Zfp69-, ZNF642-* and/or *ZNF643*-region.

In a particular preferred embodiment of the method according to the present invention, the gene for ZFP69 contains a GG to AG and/or a TT to AT heterozygosity of the mouse gene for ZFP69 and/or a T57I or A79V substitution in ZFP69 or a homologous heterozygosity and or substitution in the human gene *ZNF642* or protein ZNF642 or ZNF643. In a particular preferred embodiment of the method according to the present invention, said truncated mRNA is generated by an insertion of a DNA segment comprising a polyadenylation signal and a splicing acceptor site into intron 3 of the mouse gene for ZFP69 or a homologous insertion in the human gene *ZNF642* or *ZNF643.*

Described is a biological activity of ZFP69, ZNF642 or ZNF643 is selected from the suppression of gene expression of target genes by the inhibitory KRAB domain and/or the zinc finger DNA binding domain.

As already mentioned above, according to another aspect thereof, the object of the present invention is solved by providing a polynucleotide according to SEQ ID NO: 4, 5 or 6, a polypeptide that is encoded by SEQ ID NO: 4, 5 or 6, in particular having an amino acid sequence of SEQ ID NO: 1, 2 or 3, a polypeptide having an amino acid sequence having a 90% or more homology (as defined herein) with an amino acid sequence of SEQ ID NO: 1, 2 or 3, and exhibiting a ZFP69, ZNF642 and/or ZNF643 activity, or a polypeptide having an amino acid sequence in which 1 to 10 amino acids are deleted, substituted, and/or added in the amino acid sequence according to SEQ ID NO: 1, 2 or 3, and exhibiting a ZFP69, ZNF642 and/or ZNF643 activity for use the treatment of obesity-related diabetes, or an mammalian ortholog of these polynucleotides or polypeptides (by definition, orthologs are genes that are related by vertical descent from a common ancestor and encode proteins with the same function in different species). Further preferred are amino acid sequences or polynucleotides having 91%, 92%, 93%, 94%, 95%, 96%, 97%, 98%, 99% or 100% homology.

Described is a screening tool for an agent for treating or preventing obesity or diabetes, wherein said tool is preferably selected from a polypeptide consisting of an amino acid sequence of SEQ ID NO: 1, 2 or 3 or a mammalian ortholog of these polypeptides.

Described is a screening tool for an agent for treating or preventing obesity-related diabetes is a cell which is transformed with an expression vector comprising a polynucleotide encoding a polypeptide according to SEQ ID NO: 1, 2 or 3 or an mammalian ortholog of these polypeptides, and expresses said polypeptide. The cell can be a prokaryotic or eukaryotic cell, and the expression constructs can be present extrachromosomally or integrated into the chromosome. The polypeptide can be expressed in the form of a fusion protein, for example together with an enzymatically active moiety as reporter-construct, in order to be able to detect the expression product. Preferred host cells are derived from cells selected from the skeletal muscle, liver, adipose tissue, heart, pancreas, kidney, and/or hypothalamus.

Described is a screening tool for an agent for treating or preventing obesity or diabetes that is a non-human transgenic mammal overexpressing *Zfp69, ZNF642* or *ZNF643* and/or carrying *a Zfp69, ZNF642* or *ZNF643* genetic reporter-construct. Preferred is a transgenic mouse, rat, pig, goat or sheep, wherein the reporter-construct is preferably expressed in cells selected from the skeletal muscle, liver, adipose tissue, heart, pancreas, kidney, and/or hypothalamus of said animal. Methods to produce these non-human transgenic mammal overexpressing *Zfp69, ZNF642* or *ZNF643* and/or carrying a *Zfp69, ZNF642* or *ZNF643* genetic reporter-construct are well known to the person of skill in the art. Preferred are also transgenic non-human mammals wherein the gene that is homologous to *Zfp69*is exchanged by a gene having a modified function (e.g. knock-out or knock-in animal).

As already mentioned above, according to yet another aspect thereof, the object of the present invention is solved by providing a method of identifying a compound for treating or preventing obesity-related diabetes through modulating the expression of the full-length gene for ZFP69, ZNF642 and/or ZNF643 in a cell, comprising the steps of a) contacting a cell expressing ZFP69, ZNF642 and/or ZNF643 with at least one compound that potentially modulates the expression of ZFP69, ZNF642 and/or ZNF643 in the cell, and b) measuring the modulation of the expression of ZFP69, ZNF642 or ZNF643 in said cell. Preferred is a method according to the present invention, wherein said compound as identified is an inhibitor of the expression of ZFP69, ZNF642 or ZNF643 in said cell.

This method is suitable for the determination of compounds that can interact with ZFP69, ZNF642 or ZNF643 (or two or more of them) and to identify, for example, inhibitors, competitors or modulators of ZFP69, ZNF642 or ZNF643. Another aspect is directed at compounds that modulate the expression of *Zfp69* or *ZNF642* or *ZNF643* in a cell/in cells.

The term "contacting" in the present invention means any interaction between the potentially binding substance(s) with ZFP69, ZNF642 or ZNF643, whereby any of the two components can be independently of each other in a liquid phase, for example in solution, or in suspension or can be bound to a solid phase, for example, in the form of an essentially planar surface or in the form of particles, pearls or the like. In a preferred embodiment a multitude of different potentially binding substances are immobilized on a solid surface like, for example, on a compound library chip and ZFP69, ZNF642 or ZNF643 (or a functional part thereof) is subsequently contacted with such a chip.

The ZFP69, ZNF642 or ZNF643 employed in a method of the present invention can be a full length protein or a fragment with N/C-terminal and/or internal deletions. Preferably the fragment is either an N-terminal fragment comprising the enzymatic region of the polypeptide or a C-terminal fragment comprising the cytoplasmic region, depending on whether potentially interacting compounds are sought that specifically interact with the N- or C-terminal fragment.

The potentially binding substance, whose binding to ZFP69, ZNF642 or ZNF643 is to be measured, can be any chemical substance or any mixture thereof. For example, it can be a substance of a peptide library, a combinatory library, a cell extract, in particular a plant cell extract, a "small molecular drug", a protein and/or a protein fragment.

Measuring of binding of the compound to ZFP69, ZNF642 or ZNF643 can be carried out either by measuring a marker that can be attached either to the protein or to the potentially interacting compound. Suitable markers are known to someone of skill in the art and comprise, for example, fluorescence or radioactive markers. The binding of the two components can, however, also be measured by the change of an electrochemical parameter of the binding compound or of the protein, e.g. a change of the redox properties of either ZFP69, ZNF642 or ZNF643 or the binding compound, upon binding. Suitable methods of detecting such changes comprise, for example, potentiometric methods. Further methods for detecting and/or measuring the binding of the two components to each other are known in the art and can without limitation also be used to measure the binding of the potential interacting compound to ZFP69, ZNF642 or ZNF643 or ZFP69, ZNF642 or ZNF643 fragments. The effect of the binding of the compound to ZFP69, ZNF642 or ZNF643 can also be measured indirectly. Described are compounds that inhibit the function of the KRAB domain and/or the zinc finger DNA binding domain of ZFP69, ZNF642 or ZNF643.

As a further step after measuring the binding of a potentially interacting compound and after having measured at least two different potentially interacting compounds at least one compound can be selected, for example, on grounds of detected increase or decrease of *Zfp69, ZNF642* or *ZNF643* expression.

The thus selected binding compound is then in a preferred embodiment modified in a further step. Modification can be effected by a variety of methods known in the art, which include without limitation the introduction of novel side chains or the exchange of functional groups like, for example, introduction of halogens, in particular F, Cl or Br, the introduction of lower alkyl groups, preferably having one to five carbon atoms like, for example, methyl, ethyl, n-propyl, isopropyl, n-butyl, isobutyl, tert-butyl, n-pentyl or iso-pentyl groups, lower alkenyl groups, preferably having two to five carbon atoms, lower alkynyl groups, preferably having two to five carbon atoms or through the introduction of, for example, a group selected from the group consisting of NH₂, NO₂, OH, SH, NH, CN, aryl, heteroaryl, COH or COOH group.

The thus modified binding substances are than individually tested with a method of the present invention, i.e. they are contacted with ZFP69, ZNF642 or ZNF643 and subsequently binding of the modified compounds to the ZFP69, ZNF642 or ZNF643 polypeptide is measured. In this step, the binding per se can be measured. If needed, the steps of selecting the binding compound, modifying the binding compound, contacting the binding compound with a ZFP69, ZNF642 or ZNF643 polypeptide and measuring the binding of the modified compounds to the protein can be repeated a third or any given number of times as required. The above described method is also termed "directed evolution" since it involves a multitude of steps including modification and selection, whereby binding compounds are se lected in an "evolutionary" process optimizing its capabilities with respect to a particular property, e.g. its binding activity.

In a further embodiment of the method of the present invention, the interacting compound identified as outlined above, which may or may not have gone through additional rounds of modification and selection, is admixed with suitable auxiliary substances and/or additives. Such substances comprise pharmacological acceptable substances, which increase the stability, solubility, biocompatibility, or biological half-life of the interacting compound or comprise substances or materials, which have to be included for certain routs of application like, for example, intravenous solution, sprays, band-aids or pills.

Similar to the strategies for identifying compounds that interact with ZFP69, ZNF642 or ZNF643, compounds can be identified that modulate the expression of *Zfp69, ZNF642* or *ZNF643* in a cell. In preferred strategies, the expression of ZFP69, ZNF642 or ZNF643 can be monitored using a genetic reporter-construct for *Zfp69, ZNF642* or *ZNF643* (in order to analyse the translation efficiency and/or stability of the ZFP69, ZNF642 or ZNF643 polypeptide), for an example a fusion protein comprising a detectable fusion member (such as an enzymatic or fluorophoric group), or the amount of mRNA as present in a cell can be measured, for example, by Northern blot. The expression can also be analysed and monitored by using chip-analysis or rtPCR. Preferred compounds that modulate the expression of *Zfp69, ZNF642* or *ZNF643* in a cell are selected from specific antisense oligonucleotides, siRNAs, mRNAs or other preferably mutated nucleic acids encoding ZFP69, ZNF642 or ZNF643. These genetic elements can be used in order to provide/maintain the loss-of-function (e.g. by the truncations as identified) of *Zfp69, ZNF642* or *ZNF643* in said cell. Another preferred embodiment is the transfer of said genetic elements using gene therapy. Furthermore, encompassed are viral constructs for the introduction of said genetic elements into said cells. Alternatively, also the "naked" nucleic acid can be introduced into the cell(s), e.g. by using particle-mediated technologies. Respective methods are well described in the literature and known to the person of skill.

Described is a method for manufacturing a pharmaceutical composition for treating or preventing obesity-related diabetes, comprising the steps of: performing a screening method according to the present invention, and formulating said modulator as screened and identified into a pharmaceutical composition.

Carriers, excipients and strategies to formulate a pharmaceutical composition, for example to be administered systemically or topically, by any conventional route, in particular enterally, e.g. orally, e.g. in the form of tablets or capsules, parenterally, e.g. in the form of injectable solutions or suspensions, topically, e.g. in the form of lotions, gels, ointments or creams, or in nasal or a suppository form are well known to the person of skill and described in the respective literature.

Administration of an agent, e.g., a compound can be accomplished by any method which allows the agent to reach the target cells. These methods include, e.g., injection, deposition, implantation, suppositories, oral ingestion, inhalation, topical administration, or any other method of administration where access to the target cells by the agent is obtained. Injections can be, e.g., intravenous, intradermal, subcutaneous, intramuscular or intraperitoneal. Implantation includes inserting implantable drug delivery systems, e.g., microspheres, hydrogels, polymeric reservoirs, cholesterol matrices, polymeric systems, e.g., matrix erosion and/or diffusion systems and non-polymeric systems, e.g., compressed, fused or partially fused pellets. Suppositories include glycerin suppositories. Oral ingestion doses can be enterically coated. Inhalation includes administering the agent with an aerosol in an inhalator, either alone or attached to a carrier that can be absorbed. The agent can be suspended in liquid, e.g., in dissolved or colloidal form. The liquid can be a solvent, partial solvent or nonsolvent. In many cases, water or an organic liquid can be used.

Described is a pharmaceutical composition for treating or preventing obesity-related diabetes, obtainable by a method according to the method as above.

In certain embodiments, the compound (modulator) is administered to the subject by administering a recombinant nucleic acid. Preferably, the recombinant nucleic acid is a gene therapy vector (as also mentioned above).

Described is a method or use as above, wherein the pharmaceutical composition further comprises additional pharmaceutically active ingredients that modulate the diseases to be treated, such as obesity and diabetes.

Described is a method for treating or preventing obesity-related diabetes, comprising a diagnostic method according to the present invention as above, and providing a treatment to said mammal based on, at least in part, the result of said diagnosis. In general, the attending physician will base a treatment, and in particular obesity- and/or diabetes-preventive measures, on the diagnostic data regarding ZFP69, ZNF642 or ZNF643 of said patient, wherein said patient diagnostic data regarding ZFP69, ZNF642 or ZNF643 can be integrated with other individual patient data (clinical data, family history, DNA, etc.), and a treatment can also be performed based on the combination of these factors.

This method for example involves integrating individual diagnostic data with patient clinical information and general healthcare statistics to enable, for example, the application of personalized medicine to the mammal. Significant information about drug effectiveness, drug interactions, physiologic heart regularities and other patient status conditions could be correlated with the specific diagnostic data.

Described is a method of treating or preventing obesity-related diabetes in a mammal, comprising administering to said mammal an effective amount of a pharmaceutical composition as above. Preferably, an inhibiting active agent is administered in form of a pharmaceutical composition, such as an antibody, nucleotide or an activating binding compound. Preferably, said patient is a human being. Treating is meant to include, e.g., preventing, treating, reducing the symptoms of, or curing the disease or condition.

Described is a monoclonal antibody recognizing an epitope derived from ZFP69, ZNF642 or ZNF643 or an ortholog of this peptide sequence.

An "effective amount" is an amount of the compound(s) as mentioned above that a) acts on the expression and/or abundance of ZFP69, ZNF642 or ZNF643 as analysed, and which alleviates symptoms as found for obesity-related diabetes (which might overlap with diabetic symptoms in general). Alleviating is meant to include, e.g., preventing, treating, reducing the symptoms of, or curing the disease or condition.

Described is a method for treating a subject at risk for obesity-related diabetes, wherein a therapeutically effective amount of a modulator as above is provided. Being at risk for the disease can result from, e.g., a family history of the disease, a genotype which predisposes to the disease, or phenotypic symptoms which predispose to the disease. Described is the use of a modulator of the expression and/or the biological activity of *Zfp69, ZNF642* or *ZNF643* in a cell for the manufacture of a pharmaceutical composition for treating or preventing obesity-related diabetes. Preferred is a use, wherein said diabetes is obesity-related (as described above). More preferably, said modulator is an inhibitor of the expression of *Zfp69, ZNF642* or *ZNF643* as described herein.

The present data indicate that the diabetogenic effect of the mouse QTL Nidd1 and Nidd/SJL contributed by the NON and the SJL strains reflects allelic variance of the zinc finger domain transcription factor *Zfp69.* This allele aggravates and accelerates obesity-associated diabetes of the NZO strain, and markedly enhances hyperglycaemia of C57BL/6J-ob/ob mice. Surprisingly, diabetes appears to be produced by 'rescue' of a loss-of-function variant of *Zfp69*: NZO and the diabetes-resistant C57BL/6J strain express a truncated mRNA, whereas 'normal' expression was induced by the diabetogenic allele from SJL and NON. The following arguments can be made in favour of these conclusions:
1. With interval-specific congenics, the inventors defined a critical genomic interval with 11 genes that was required to enhance diabetes in NZO,
2. of the 11 genes located in that interval only *Zfp69*_exhibited a significant functional allelic variance,
3. this allelic variance corresponded with the diabetogenic or diabetes-resistant effect of the chromosome 4 QTL in 5 mouse strains, and
4. the gene expression profile of *Zfp69* (liver, adipose tissue, pancreas) is consistent with the observed in-vivo effects of Zfp^{SJL}.

Thus, the loss-of-function variant Zfp^{6J} appears to be a major contributor to the diabetes resistance of the C57BL/6J strain [Herberg and Leiter, 2001], and expression of Zfp^{SJL} causes a 'dose dependent' diabesity.

In order to elucidate the mechanism the diabetogenic effect of Zfp^{SJL}, the inventors have studied C57BL/6J-ob/ob-Zfp^{SJL} mice. Because of their monogenic obesity, these mice are much more homogeneous than the polygenic NZO x SJL outcross population. The effects observed in C57BL/6J-ob/ob-Zfp^{SJL} mice are consistent with the conclusion that Zfp^{SJL} enhanced insulin resistance by reduction of serum adiponectin and accumulation of liver triglycerides. Interestingly, the Zfp^{SJL} genotype was associated with a markedly increased expression of Cidea in liver. Knockout mice of Cidea have previously been shown to be obesity and diabetes resistant [Zhou et al., 2003]. Surprisingly, the Zfp^{SJL} genotype was also associated with reduced islet area, reduced pancreatic insulin content, and relative hypoinsulinemia. Thus, the effects of Zfp^{SJL} are pleiotropic, and the transcription factor appears to be a key regulator of pathways involved in glucose and lipid metabolism.

It should be noted that the diabetogenic effect of the *Zfp69* variant seemed to be dependent on interaction with other genes contributed by the background strain. *Zfp69* required obesity in order to produce hyperglycaemia ('diabesity'), and needs other diabetogenic alleles in order to produce beta cell destruction, hypoinsulinaemia and severe diabetes. So far, the inventors could not detect beta cell destruction on the C57BL/6J background. On the NZO background, it was the interaction with NZO alleles on chromosomes 1 and 15 that enhanced the diabetogenic effect of *Zfp69.*

So far, the precise molecular and cellular function of *Zfp69* is unknown. From its sequence homology it appears safe to conclude that the gene belongs to a family of transcription factors that comprise the conserved Krüppel-associated box (KRAB) in addition to the zinc finger DNA binding domain [Bellefroid et al., 1991; Urrutia, 2003]. The KRAB domain appears to activate co-repressors, resulting in a suppression of gene expression of target genes [Peng et al., 2007]. Such a mechanism could explain the reduced expression of PPARa target genes and of adiponectin in Zfp^{SJL} mice. However, given that Zfp^{SJL} controls gene expression exclusively through its inhibitory KRAB domain, the markedly enhanced expression of Cidea requires assumption of an intermediate inhibitor which is suppressed by Zfp^{SJL}.

The production of an aberrant mRNA by alternative splicing is common in human inherited disease. Here, the inventors have identified a so far unusual mechanism: a truncated Zfp69 mRNA was generated by insertion of a DNA segment comprising a polyadenylation signal and a splicing acceptor site into intron 3. By a similar mechanism, expression of the endothelin B receptor is reduced in piebald mice [Yamada et al., 2006]. Furthermore, it was shown recently that human soluble VEGF receptor is generated, and its abundance regulated, by intronic polyadenylation and alternative splicing [Thomas et al., 2007].

The following figures, sequences, and examples merely serve to illustrate the invention and should not be construed to restrict the scope of the invention to the particular embodiments of the invention described in the examples. All references cited in the text are hereby incorporated in their entirety by reference.

SEQ ID NOs 1, 2 and 3 show the polypeptide sequences of the mouse protein ZFP69 (Genbank Acc No. NM_001005788) and the human protein ZNF642 (Genbank Acc No. NM_198494) and ZNF643 (Genbank Acc No. NM_023070), respectively.

SEQ ID NOs 4, 5 and 6 show nucleotide sequence of the mouse gene for ZFP69 (Genbank Acc No. NM_001005788) and the human gene for ZNF642 (Genbank Acc No. NM_198494), and ZNF643 (Genbank Acc No. NM_023070), respectively.

### Figures

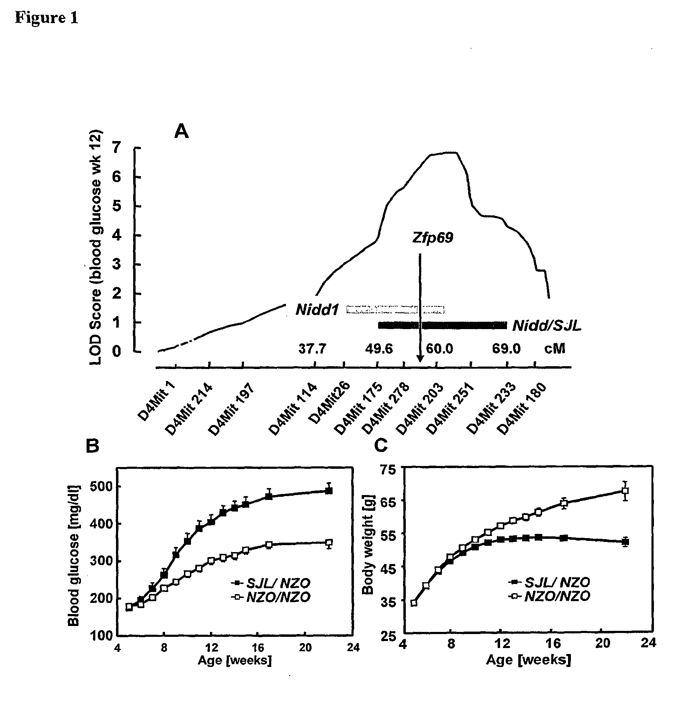

Figure 1 shows the location and diabetogenic effect of QTL Nidd/SJL on distal mouse chromosome 4. A: LOD score curve of Nidd/SJL derived from a NZO x (NZO x SJL) backcross population of 207 mice. The approximate position of QTL Nidd1 harbouring a diabetogenic allele from NON was obtained from Leiter et al. (1998). B and C: The chromosomal segment D4Mit175-D4Mit233 of SJL was introgressed on a C57BL/6J background, and a N2 (NZO x Nidd/SJL-C57BL/6J) backcross population was generated. Blood glucose (B) and weight gain (C) in backcross mice were monitored weekly. Data represent means +/- SE.

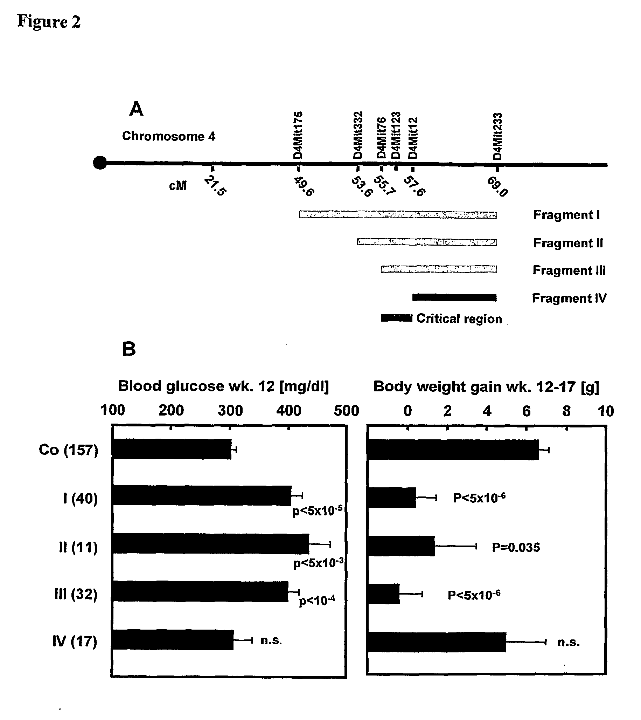

Figure 2 shows the identification of a critical interval of mouse chromosome 4 harbouring the diabetogenic allele Nidd/SJL. A: Map of chromosomal fragments I-IV of SJL carried by recombinant congenic lines generated on the C57BL/6J background. B: Blood glucose (wk 12) and weight gain (wk 12-17) in mice from N2 (NZO x Nidd/SJL-B6) backcross mice generated with the recombinant congenic lines. Differences to controls (complete fragment I homozygous for the NZO genotype) were determined by t-test. The number of mice in each group is given in parenthesis.

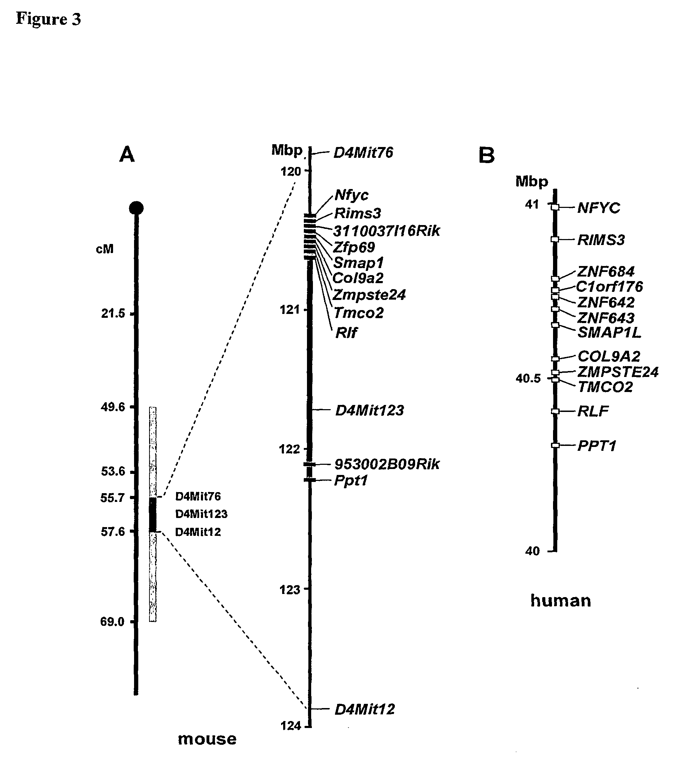

Figure 3 shows the map of the critical segment of Nidd/SJL.as defined by the markers, D4Mit76, D4Mit123, and D4Mit12, and further fine-mapped to a region between the genes Nfyk and Ppt1 (red segment). B: Comparison of the segment with the syntenic human region.
Figure 4 shows the generation of a loss-of-function mRNA of *Zfp69* by intronic polyadenylation and alternative splicing. A: Genomic organisation and PCR primer pairs used for characterisation of *Zfp69.* B: Sequence of the additional exon 3A that was polyadenylated and spliced to exon 3, as identified by 3'-RACE-PCR. C, D: Detection of the cDNA variants in B6 and SJL by PCR with downstream primers derived from exons 3A and 4. F,G: Allelic variation of *Zfp69* in different mouse strains with known contribution of distal chromosome 4 to diabesity. H: Model of the domain structure of ZFP69 and of its truncated variant (B6, NZO).

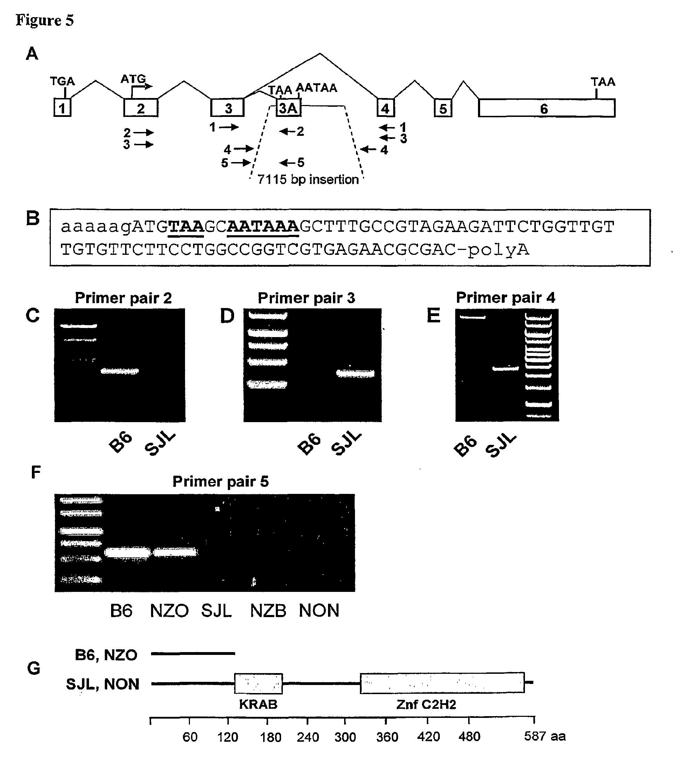

Figure 5 shows the loss-of-function of *Zfp69* in NZO and C57BL/6J (B6) by alternative mRNA splicing and intronic polyadenylation. A: Genomic organisation and PCR primer pairs used for characterisation of *Zfp69.* B: Sequence of the additional exon 3A (capital letters) that was polyadenylated and spliced to exon 3, as identified by 3'-RACE-PCR. C, D: Identification of the cDNA variants in B6 and SJL by PCR with downstream primers derived from exons 3A and 4. E, F: Detection of the 7 kbp insertion in intron 3 of B6 (E) and allelic variation of *Zfp69* in different mouse strains with known contribution of distal chromosome 4 to diabesity. G: Model of the domain structure of ZFP69 and of its truncated variant (B6, NZO).

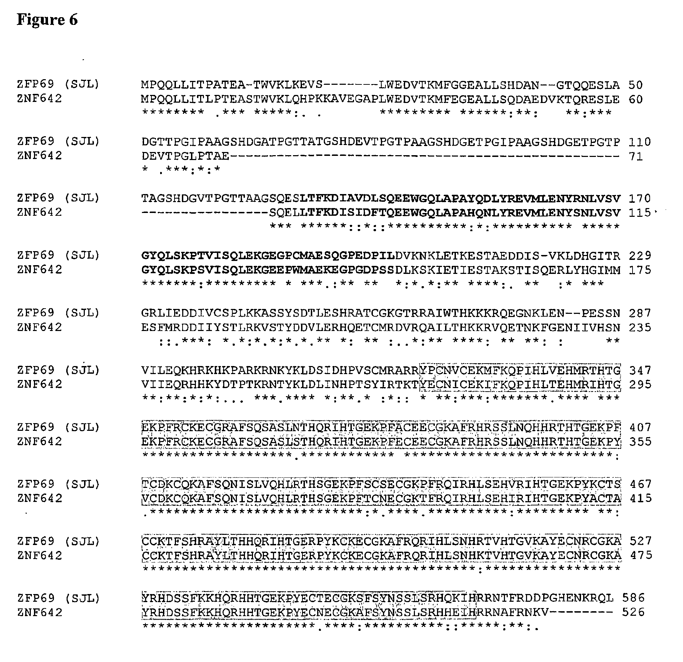

Figure 6 shows the sequence comparison of ZFP69 (mouse) and ZNF642 (human), bold letters indicate the KRAB-domain; the light gray background indicates the zinc finger domain.

### Examples

Identification and fine-mapping of a critical diabetogenic interval of Nidd/SJL - Fig. 1A illustrates the position of the Nidd/SJL locus on distal chromosomes 4 [Plum et al., 2000, 2002] and its proximity to the previously described Nidd1 [Leiter et al., 1998]. For further isolation and fine-mapping of the locus, the inventors generated interval-specific congenic mice by introgressing the indicated segment between the markers D4Mit175 and D4Mit233 into the C57BL/6J background. In a parallel outcross experiment of C57BL/6J with NZO the inventors ascertained that there was no diabetogenic effect from chromosome 4 of C57BL/6J. In order to introduce obesity, congenic C57BL/6J mice carrying Nidd/SJL (SB6) were then mated with NZO, and the resulting F1 was intercrossed or backcrossed on NZO. Characterisation of the N2 progeny indicated that Nidd/SJL carriers exhibited severe hyperglycaemia with higher blood glucose levels approximately 150 mg/dl higher than in carriers of the C57BL/6J allele (Fig. 1B), and stopped gaining weight at the onset of diabetes (week 10-12, Fig. 1C). Similar results were obtained in the F2 intercross which showed an additive effect of Nidd/SJL. Histology of the pancreas from carriers of Nidd/SJL indicated that hyperglycaemia was associated with a progredient loss of beta cells. It should be noted that less severe hyperglycaemia also developed in non-carriers of Nidd/SJL (Fig. 1B) due to other diabetogenic alleles from NZO chromosomes 1 and 15. These mice, however, continued gaining weight (Fig. 1C), indicating that the weight development could be used as an additional criterion to determine the presence or absence of Nidd/SJL.

For identification of a smaller critical segment of Nidd/SJL, congenic SJL- C57BL/6J mice carrying the indicated fragments of the QTL (Fig. 2A) were mated to NZO and backcrossed. Characterisation of the N2 progeny with regard to their blood glucose levels and development of body weight indicated that segments I, II and III were diabetogenic (Fig. 2B and C). In contrast, introgression of segment IV, which serendipitously originated from segment III during amplification of breeding partners, into the NZO background failed to produce the severe hyperglycaemia and growth arrest. Thus, a critical interval of chromosome 4 that comprised the diabetogenic allele was defined by the markers D4Mit76, D4Mit123 and D4Mit12. For further fine mapping the inventors used additional SNPs from the public databases, thereby reducing the critical interval defined by the genotypes of segments III and IV to approximately 2 Mbp (Fig. 3). The interval was flanked by Nfyc and Ppt1, and contained 11 confirmed genes and 9 LOC sequences. None of the LOC sequences exhibited significant similarities with other human or mouse genes, ESTs or genomic sequences in other species. The syntenic human region contained two additional genes (Fig. 3B; *ZNF684* and *ZNF643),* presumably duplications of *ZNF642.* Data base searches indicated that these genes were not present in the mouse genome; their closest mouse isolog is *Zfp69.*

Identification of a loss-of-function variant of *Zfp69 -* Sequencing of exons of all 11 genes in the critical interval identified two substitutions in *Zfp69* (T57I, A79V), two three conservative amino acid exchanges in the sequence of the collagen Co19A2 (T298I, A482I, R610H) and one substitution in Smap11 (T257I). In addition, the inventors determined the expression of all 11 confirmed genes in liver, adipose tissue, skeletal muscle and pancreas from SJL and NZO by quantitative PCR. The only differentially expressed gene in the region was *Zfp69.* As is illustrated in Fig. 4, the inventors detected a marked difference in its expression between the two strains NZO and SJL: in all tissues investigated, mRNA levels of *Zfp69* were essentially undetectable in NZO, and 4-8 fold higher in SJL (Fig. 4A). Analysis of tissues from SB6-ob/ob mice congenic for Nidd/SJL indicated that the expression of *Zfp69* was dependent on the genotype of the critical region on chromosome 4 (Fig. 4B).

In order to test the possibility that the marked difference in mRNA levels between NZO and SJL reflected the presence of different mRNA species, the inventors further analysed the *Zfp69* cDNA by RACE-PCR in order to detect splicing variants and/or alternative transcription starts. Products of 5'-RACE were identical in the two strains. By 3'-RACE, however, the inventors detected a shorter cDNA in C57BL/6J that contained only the first three exons fused to a short segment of intron 3 (Fig. 5B; alternative exon 3A in Fig. 5A); this segment comprised a stop codon and a polyadenylation site. PCR with a primer matching exon 3A indicated that the shorter cDNA was exclusively expressed in C57BL/6J (Fig. 5C). Conversely, the full length cDNA of ZFP69 comprising exon 4 was nearly undetectable in C57BL/6J (Fig. 5D). In order to identify the sequence variation responsible for the marked difference between NZO and SJL, intron 3 of SJL was further characterised by PCR. Comparison with the reference sequence (C57BL/6J) revealed the presence of a deletion of the sequence harbouring exon 3A and the polyadenylation sites in SJL (Fig. 5E).

ZFP69 is a member of the subfamily of zinc finger transcription factors that comprise an N-terminal KRAB and a zinc finger binding C2-H2 domain (Fig. 5H). The shorter mRNA generated in B6 and NZO encodes a truncated protein which can be considered a loss-of-function variant, since it lacks both the KRAB and the (DNA binding) C2H2 domain of the transcription factor.

Allelic variation of *Zfp69* in mouse strains NZO, B6, NZB, SJL, and NON corresponds with expression of *Zfp69* and with the diabetogenic effect of chromosome 4 in 3 outcross populations - Several outcross experiments generating obese mouse populations have previously been performed that showed the presence or absence of a diabetogenic allele in the vicinity of D4Mit228 on chromosome 4: NZO-NON (Leiter et al., 1998), NZOSJL (Plum et al., 2000), NZO-C57BL/6J, and NZO-NZB. According to these data, NON and SJL contributed a major diabetogenic effect (Niddl and Nidd/SJL). In addition, distal chromosome 4 of NZB (D4Mit203) contributed to the hyperglycemia of the NZO-NZB F2 (blood glucose in wk 22: genotype NZO/NZO, 301 +/- 32; NZO/NZB, 389 +/- 19; NZB/NZB, 376 +/- 28 mg/dl; p<0.05 for differences to NZO/NZO). Based on these data, the inventors expected that SJL, NON and NZB carry an identical (diabetogenic) allele of Zfp69, and that NZO and B6 both carry the diabetes-protecting allele. Indeed, the contribution of the different mouse strain to the hyperglycaemia in the outcross populations corresponded with the allelic variation of *Zfp69* in these strains (Figs. 5F), and with the expression of the full-length mRNA (Fig. 5G). These data are consistent with the conclusion that expression of the full-length *Zfp69* on an obese background is diabetogenic, whereas expression of the truncated variant protects from diabetes.

Characterisation of C57BL/6J-ob/ob mice carrying the diabetogenic *Zfp69* allele - In order to define the functional effects of the diabetogenic *Zfp69* allele, the inventors characterized C57BL/6J-ob/ob-*Zfp69*^{SJL/SJL} in comparison with C57BL/6J -ob/ob-*Zfp69*^{6J/6J} mice. These mice exhibited an identical time course of weight gain, thereby excluding that any of the observed effects reflected differences in the degree of obesity between the genotypes. As anticipated, mice carrying *Zfp69*^{SJL/SJL} exhibited fasting hyperglycaemia that was not associated with increased insulin levels. Morphometric analysis of the pancreas revealed a significantly reduced islet area in *Zfp69*^{SJL/SJL} carriers that was paralleled by a proportional reduction in insulin content. In addition, the SJL genotype was associated with a significantly increased liver fat content. These data suggest that the transcription factor exerts pleiotropic effects in beta cells, liver, and adipose tissue.

### List of references as cited

Bellefroid EJ, Poncelet DA, Lecocq PJ, Revelant O, Martial JA. The evolutionarily conserved Kruppel-associated box domain defines a subfamily of eukaryotic multifingered proteins. Proc Natl Acad Sci U S A 1991; 88:3608-3612
Clee SM, Yandell BS, Schueler KM, Rabaglia ME, Richards OC, Raines SM, Kabara EA, Klass DM, Mui ET, Stapleton DS, Gray-Keller MP, Young MB, Stoehr JP, Lan H, Boronenkov I, Raess PW, Flowers MT, Attie AD. Positional cloning of Sorcs1, a type 2 diabetes quantitative trait locus. Nat Genet 2006; 38:688-693
Florez JC, Hirschhorn J, Altshuler D. The inherited basis of diabetes mellitus: implications for the genetic analysis of complex traits. Annu Rev Genomics Hum Genet 2003; 4:257-291
Herberg L, Leiter EH: Obesity/diabetes in mice with mutations in the leptin or leptin receptor genes. In: Frontiers in animal diabetes research, Vol. 2: Animal models of diabetes, pp 63-107. Eds. Sima AAF, Shafrir, E; harwood academic publishers, Amsterdam 2001
Herberg L, Coleman DL: Laboratory animals exhibiting obesity and diabetes syndromes. Metabolism 1977; 26:59-98
Kahn BB. Type 2 diabetes: when insulin secretion fails to compensate for insulin resistance. Cell 1998; 92:593-596
Leiter EH, Reifsnyder PC, Flurkey K, Partke HJ, Junger E, Herberg L: NIDDM genes in mice. Deleterious synergism by both parental genomes contributes to diabetic thresholds. Diabetes 1998; 47:1287-1295
Peng H, Gibson LC, Capili AD, Borden KL, Osborne MJ, Harper SL, Speicher DW, Zhao K, Marmorstein R, Rock TA, Rauscher FJ 3rd. The structurally disordered KRAB repression domain is incorporated into a protease resistant core upon binding to KAP-1-RBCC domain. J Mol Biol 2007; 370:269-289
Plum L, Giesen K, Kluge R, Junger E, Linnartz K, Schürmann A, Becker W, Joost HG: Characterization of the diabetes susceptibility locus Nidd/SJL in the New Zealand obese (NZO) mouse: Islet cell destruction, interaction with the obesity QTL Nobl, and effect of dietary fat. Diabetologia 2002; 45: 823-830
Plum L, Kluge R, Giesen K, Altmüller J, Ortlepp JR, Joost HG: Type-2-diabetes-like hyperglycemia in a backcross model of New Zealand obese (NZO) and SJL mice: Characterization of a susceptibility locus on chromosome 4 and its relation with obesity. Diabetes 2000; 49: 1590-1596
Reifsnyder PC, Leiter EH: Deconstructing and reconstructing obesity-induced diabetes (diabesity) in mice. Diabetes 2002; 51: 825-832
Saxena R, et al.: Genome-wide association analysis identifies loci for type 2 diabetes and triglyceride levels. Science 2007; 316:1331-1336
Scott LJ, et al.: A genome-wide association study of type 2 diabetes in Finns detects multiple susceptibility variants. Science 2007; 316:1341-1345
Sladek R, et al.: A genome-wide association study identifies novel risk loci for type 2 diabetes. Nature 2007; 445:881-885
Steinthorsdottir V, et al.: A variant in CDKAL1 influences insulin response and risk of type 2 diabetes. Nat Genet 2007; 39:770-775
Thomas CP, Andrews JI, Liu KZ. Intronic polyadenylation signal sequences and alternate splicing generate human soluble Fltl variants and regulate the abundance of soluble Flt1 in the placenta. FASEB J 2007 Jul 5
Thompson DB, Janssen RC, Ossowski VM, Prochazka M, Knowler WC, Bogardus C. Evidence for linkage between a region on chromosome 1p and the acute insulin response in Pima Indians. Diabetes 1995; 44:478-81
Urrutia R. KRAB-containing zinc-finger repressor proteins. Genome Biol 2003; 4:231 Yamada T, Ohtani S, Sakurai T, Tsuji T, Kunieda T, Yanagisawa M. Reduced expression of the endothelin receptor type B gene in piebald mice caused by insertion of a retroposon-like element in intron 1. J Biol Chem 2006; 281:10799-807
Zeggini E, et al.; Wellcome Trust Case Control Consortium (WTCCC), McCarthy MI, Hattersley AT: Replication of genome-wide association signals in UK samples reveals risk loci for type 2 diabetes. Science 2007; 316:1336-1341
Zhou Z, Yon Toh S, Chen Z, Guo K, Ng CP, Ponniah S, Lin SC, Hong W, Li P. Cidea-deficient mice have lean phenotype and are resistant to obesity. Nat Genet 2003; 35:49-56

## Claims

1. A method for diagnosing obesity-related diabetes or an increased risk for obesity-related diabetes in a mammal, comprising
- analyzing the expression of the full-length gene for mammalian ZPF69, preferably for human ZNF642 and/or human ZNF643, in a biological sample obtained from a mammal to be diagnosed,
wherein a lack or reduced expression of the full-length gene for ZFP69, ZNF642 and/or ZNF643 in said mammal, when compared to a biological sample obtained from a non-diseased mammal, is indicative for a normal or for a reduced risk for obesity-related diabetes in said mammal.

2. The method according to claim 1, wherein said mammal is a mouse, rat or human, and/or wherein said biological sample contains biological material that is derived from liver, skeletal muscle, and/or adipose tissue.

3. The method according to claim 1 or 2, further comprising using ZFP69-, ZNF64- and/or ZNF643-specific antibodies, hybridization analysis, quantitative PCR, mRNA analysis, quantitative analysis of the amount of ZFP69-, ZNF642- and/or ZNF643-protein, and/or sequencing of the *Zfp69-, ZNF642-* and/or *ZNF643*-locus*.*

4. The method according to any of claims 1 to 3, further comprising analyzing the truncation of the mRNA of *Zfp69, ZNF642* and/or *ZNF643* and/or analyzing SNPs in the chromosomal *Zfp69-, ZNF642-* and/or *ZNF643-*region*.*

5. The method according to any of claims 1 to 4, wherein the gene for ZFP69 contains a GG to AG and/or a TT to AT heterozygosity of the mouse gene for ZFP69 and/or a T57I or A79V substitution in ZFP69 or a homologous heterozygosity and/or a substitution in the human gene *ZNF642* or protein ZNF642 or ZNF643.

6. The method according to any of claims 4 or 5, wherein said truncated mRNA is generated by an insertion of a DNA segment comprising a polyadenylation signal and a splicing acceptor site into intron 3 of the mouse gene for ZFP69 or a homologous insertion in the human gene *ZNF642* or *ZNF643.*

7. A polynucleotide according to SEQ ID NO: 1, 2 or 3,
a polypeptide that is encoded by SEQ ID NO: 1, 2 or 3, in particular having an amino acid sequence of SEQ ID NO: 4, 5 or 6,
a polypeptide having an amino acid sequence having a homology of 90 % or more with an amino acid sequence of SEQ ID NO: 4, 5 or 6, and exhibiting a ZFP69, ZNF642 and/or ZNF643 activity, or
a polypeptide having an amino acid sequence in which 1 to 10 amino acids are deleted, substituted, and/or added in the amino acid sequence according to SEQ ID NO: 4, 5 or 6, and exhibiting a ZFP69, ZNF642 and/or ZNF643 activity,
for use in the treatment of obesity-related diabetes.

8. Use of a polynucleotide or a polypeptide selected from the group consisting of
a) a polynucleotide according to SEQ ID NO: 1, 2 or 3,
b) a polypeptide that is encoded by SEQ ID NO: 1, 2 or 3, in particular having an amino acid sequence of SEQ ID NO: 4, 5 or 6,
c) a polypeptide consisting of an amino acid sequence of SEQ ID NO: 4, 5 or 6,
d) a polypeptide having an amino acid sequence having a homology of 90 % or more with an amino acid sequence of SEQ ID NO: 4, 5 or 6, and exhibiting a ZFP69, ZNF642 and/or ZNF643 activity, and
e) a polypeptide having an amino acid sequence in which 1 to 10 amino acids are deleted, substituted, and/or added in the amino acid sequence according to SEQ ID NO: 4, 5 or 6, and exhibiting a ZFP69, ZNF642 and/or ZNF643 activity as a screening tool for an agent for treating or preventing obesity-related diabetes, wherein the screening tool is not used in a human beeing.

9. An *in vitro* method of identifying a compound for treating or preventing obesity-related diabetes through modulating the expression of the full-length gene for ZFP69, preferably for human *ZNF642* and/or for human ZNF643 in a cell, comprising the steps of
a) contacting a cell expressing ZFP69, ZNF642 and/or ZNF643 with at least one compound that potentially modulates the expression of ZFP69, ZNF642 and/or ZNF643 in the cell, and
b) measuring the modulation, preferably the inhibition, of the expression of *Zfp69, ZNF642* and/or *ZNF643* in said cell,
wherein said compound is preferably selected from a peptide library, a combinatory library, a cell extract, in particular a plant cell extract, a "small molecular drug", an antisense oligonucleotide, an siRNA, an mRNA or nucleic acid encoding *Zfp69, ZNF642* and/or *ZNF643* or a mutated form thereof, and an antibody or fragment thereof specifically recognizing the amino acid sequence of ZFP69, ZNF642 and/or ZNF643.

10. The method according to claim 9, wherein said cell is selected from the group consisting of cells of the skeletal muscle, liver, adipose tissue, heart, pancreas, kidney, and hypothalamus.

## Patentansprüche

1. Verfahren zur Diagnose von mit Fettsucht zusammenhängender Diabetes oder einem erhöhten Risiko für mit Fettsucht zusammenhängender in einem Säugetier, umfassend
- Analysieren der Expression des Vollängengens für Säugetier-ZFP69, bevorzugt für menschliches ZNF642 und/oder menschliches ZNF643 in einer biologischen Probe, die von einem zu diagnostizierenden Säugetier erhalten wurde,
wobei ein Fehlen oder die verringerte Expression des Vollängengens für ZFP69, ZNF642 und/oder ZNF643 in dem Säugetier, wenn verglichen mit einer biologischen Probe, die von einem nicht erkrankten Säugetier erhalten wurde, ein normales oder reduziertes Risiko für mit Fettsucht zusammenhängender Diabetes in dem Säugetier anzeigt.

2. Verfahren nach Anspruch 1, wobei das Säugetier eine Maus, Ratte oder Mensch ist und/oder wobei die biologischen Probe biologisches Material enthält, das von Leber, Skelettmuskulatur und/oder Fettgewebe abgeleitet ist.

3. Verfahren nach Anspruch 1 oder 2, weiter umfassend die Verwendung von ZFP69-, ZNF64- und/oder ZNF643-spezifischen Antikörpern, Hybridisierungs-Analyse, quantitativer PCR, mRNA Analyse, quantitativer Analyse der Menge an ZFP69-, ZNF642- und/oder ZNF643-Protein, und/oder Sequenzieren des *Zfp69-, ZNF642-* und/oder *ZNF643*-Lokus.

4. Verfahren nach einem der Ansprüche 1 bis 3, weiter umfassend Analysieren der Verkürzung der mRNA von *Zfp69, ZNF642* und/oder *ZNF643* und/oder Analysieren von SNPs in der chromosomalen *Zfp69-, ZNF642-* und/oder ZNF643-Region.

5. Verfahren nach einem der Ansprüche 1 bis 4, wobei das Gen für ZFP69 eine GG zu AG und/oder eine TT zu AT Heterozygosität des Maus-Gens für ZFP69 und/oder eine T57I oder A79V Substitution in ZFP69 oder eine homologe Heterozygosität und/oder eine Substitution in dem menschlichen Gen *ZNF642* oder Protein ZNF642 oder ZNF643 enthält.

6. Verfahren nach einem der Ansprüche 4 oder 5, wobei die verkürzte mRNA durch eine Insertion eines DNA Segments umfassend ein Polyadenylierungssignal und eine Splicing-Akzeptorstelle in Intron 3 des Maus-Gens für ZFP69 oder eine homologe Insertion im menschlichen Gen *ZNF642* oder *ZNF643* erzeugt wird.

7. Polynukleotid nach SEQ ID NO: 1, 2 oder 3,
Polypeptid, das durch SEQ ID NO: 1, 2 oder 3 kodiert wird, das insbesondere eine Aminosäuresequenz von SEQ ID NO: 4, 5 oder 6 aufweist,
Polypeptid, das eine Aminosäuresequenz aufweist, die eine Homologie von 90 % oder mehr zu einer Aminosäuresequenz von SEQ ID NO: 4, 5 oder 6 aufweist, und eine ZFP69-, ZNF642- und/oder ZNF643-Aktivität zeigt, oder
Polypeptid, das eine Aminosäuresequenz aufweist, in der 1 bis 10 Aminosäuren deletiert, substituiert und/oder hinzugefügt sind in der Aminosäuresequenz nach SEQ ID NO: 4, 5 oder 6 und das eine ZFP69-, ZNF642- und/oder ZNF643-Aktivität zeigt, zur Verwendung in der Behandlung von mit Fettsucht zusammenhängender Diabetes.

8. Verwendung eines Polynukleotids oder eines Polypeptids ausgewählt aus der Gruppe bestehend aus
a) einem Polynukleotid nach SEQ ID NO: 1, 2 oder 3,
b) einem Polypeptid, das durch SEQ ID NO: 1, 2 oder 3 kodiert wird, das insbesondere eine Aminosäuresequenz von SEQ ID NO: 4, 5 oder 6 aufweist,
c) einem Polypeptid bestehend aus einer Aminosäuresequenz nach SEQ ID NO: 4, 5 oder 6,
d) einem Polypeptid, das eine Aminosäuresequenz aufweist, die eine Homologie von 90 % oder mehr zu einer Aminosäuresequenz von SEQ ID NO: 4, 5 oder 6 aufweist, und eine ZFP69-, ZNF642- und/oder ZNF643-Aktivität zeigt, und
e) einem Polypeptid, das eine Aminosäuresequenz aufweist, in der 1 bis 10 Aminosäuren deletiert, substituiert und/oder hinzugefügt sind in der Aminosäuresequenz nach SEQ ID NO: 4, 5 oder 6 und das eine ZFP69-, ZNF642-und/oder ZNF643-Aktivität zeigt
als ein Screeningtool für ein Mittel zur Behandlung oder der Prävention von mit Fettsucht zusammenhängender Diabetes, wobei das Screeningtool nicht in einem Menschen verwendet wird.

9. *In vitro*-Verfahren zur Identifizierung einer Verbindung zur Behandlung oder der Prävention von mit Fettsucht zusammenhängender Diabetes durch Modulieren der Expression des Vollängengens für ZFP69, bevorzugt für menschliches ZNF642 und/oder für menschliches ZNF643 in einer Zelle, umfassend die Schritte von
a) in Kontakt einer Zelle, die ZFP69, ZNF642 und/oder ZNF643 exprimiert, mit mindestens einer Verbindung, die potentiell die Expression von ZFP69, ZNF642 und/oder ZNF643 in der Zelle moduliert, und
b) Messen der Modulation, bevorzugt der Inhibierung, der Expression von *Zfp69, ZNF642* und/oder *ZNF643* in der Zelle,
wobei die Verbindung bevorzugt ausgewählt ist aus einer Peptidbibliothek, einer kombinatorischen Bibliothek, einem Zellextrakt, insbesondere einem Planzenzellextrakt, einem Wirkstoff mit kleinem Molekulargewicht, einem antisense-Oligonukleotid, einer siRNA, einer mRNA oder Nukleinsäure, die für *Zfp69, ZNF642* und/oder *ZNF643* oder eine mutierte Form davon kodiert, und ein Antikörper oder Fragment davon, der/das spezifisch die Aminosäuresequenz von ZFP69, ZNF642 und/oder ZNF643 erkennt.

10. Verfahren nach Anspruch 9, wobei die Zelle ausgewählt ist aus der Gruppe bestehend aus Zellen der Skelettmuskulatur, Leber, Fettgewebe, Herz, Pankreas, Niere und Hypothalamus.

## Revendications

1. Procédé pour le diagnostic du diabète lié à l'obésité ou d'un risque accru de diabète lié à l'obésité chez un mammifère, comprenant
- l'analyse de l'expression génique dans son intégralité pour le gène ZFP69 de mammifère, de préférence pour le ZNF642 humain et/ou le ZNF643 humain, dans un échantillon biologique prélevé chez un mammifère devant recevoir un diagnostic,
dans lequel une absence ou une diminution de l'expression génique dans son intégralité pour le(s) gène(s) ZFP69, ZNF642 et/ou ZNF643 chez ledit mammifère, en comparaison avec un échantillon biologique prélevé chez un mammifère sain, indique un risque normal ou réduit de diabète lié à l'obésité chez ledit mammifère.

2. Procédé conforme à la revendication 1, dans lequel ledit mammifère est une souris, un rat ou un être humain, et/ou dans lequel ledit échantillon biologique contient une substance biologique provenant du foie, du muscle squelettique et/ou du tissu adipeux.

3. Procédé conforme à la revendication 1 ou 2, comprenant également l'utilisation d'anticorps spécifiques de ZFP69, ZNF64 et/ou ZNF643, une analyse d'hybridation, une PCR quantitative, une analyse de l'ARNm, une analyse quantitative de la quantité de protéines codant pour ZFP69, ZNF642 et/ou ZNF643 et/ou le séquençage du locus *Zfp69, ZNF642* et/ou *ZNF643.*

4. Procédé conforme à l'une des revendications 1 à 3, comprenant également l'analyse de la troncature de l'ARNm de *Zfp69, ZNF642* et/ou *ZNF643* et/ou l'analyse des SNP dans la région chromosomique *Zfp69, ZNF642* et/ou *ZNF643.*

5. Procédé conforme à l'une des revendications 1 à 4, dans lequel le gène ZFP69 contient une hétérozygotie GG à AG et/ou TT à AT du gène de souris ZFP69 et/ou une substitution T57I ou A79V dans ZFP69 ou une hétérozygotie homologue et/ou une substitution dans le gène humain *ZNF642* ou la protéine ZNF642 ou ZNF643.

6. Procédé conforme à la revendication 4 ou 5, dans lequel ledit ARNm tronqué est généré par une insertion d'un segment d'ADN comprenant un signal de polyadénylation et un site accepteur d'épissage dans l'intron 3 du gène de souris ZFP69 ou une insertion homologue dans le gène humain *ZNF642* ou *ZNF643.*

7. Polynucléotide conforme à la séquence SEQ ID n° 1, 2 ou 3,
polypeptide codé par la séquence SEQ ID n° 1, 2 ou 3, en particulier ayant une séquence d'acides aminés de SEQ ID n° 4, 5 ou 6,
polypeptide ayant une séquence d'acides aminés présentant une homologie de 90 % ou plus avec une séquence d'acides aminés de SEQ ID n° 4, 5 ou 6, et présentant une activité ZFP69, ZNF642 et/ou ZNF643, ou
polypeptide ayant une séquence d'acides aminés dans laquelle 1 à 10 acides aminés sont délétés, substitués et/ou ajoutés dans la séquence d'acides aminés conforme à la séquence SEQ ID n° 4, 5 ou 6, et présentant une activité ZFP69, ZNF642 et/ou ZNF643,
pour utilisation dans le traitement du diabète lié à l'obésité.

8. Utilisation d'un polynucléotide ou d'un polypeptide sélectionné à partir du groupe consistant en
a) un polynucléotide conforme à la séquence SEQ ID n° 1, 2 ou 3,
b) un polypeptide codé par la séquence SEQ ID n° 1, 2 ou 3, en particulier ayant une séquence d'acides aminés de SEQ ID n° 4, 5 ou 6,
c) un polypeptide consistant en une séquence d'acides aminés de SEQ ID n° 4, 5 ou 6,
d) un polypeptide ayant une séquence d'acides aminés présentant une homologie de 90 % ou plus avec une séquence d'acides aminés de SEQ ID n° 4, 5 ou 6, et présentant une activité ZFP69, ZNF642 et/ou ZNF643, et
e) un polypeptide ayant une séquence d'acides aminés dans laquelle 1 à 10 acides aminés sont délétés, substitués et/ou ajoutés dans la séquence d'acides aminés conforme à la séquence SEQ ID n° 4, 5 ou 6, et présentant une activité ZFP69, ZNF642 et/ou ZNF643
comme outil de dépistage pour un agent destiné à traiter ou à prévenir le diabète lié à l'obésité, dans lequel l'outil de dépistage n'est pas utilisé chez l'être humain.

9. Procédé *in vitro* pour l'identification d'un composé visant à traiter ou à prévenir le diabète lié à l'obésité par le biais de la modulation de l'expression génique dans son intégralité pour le gène ZFP69, de préférence pour le ZNF642 humain et/ou le ZNF643 humain dans une cellule, comprenant les étapes suivantes :
a) mettre en contact une cellule exprimant ZFP69, ZNF642 et/ou ZNF643 avec au moins un composé qui module potentiellement l'expression de ZFP69, ZNF642 et/ou ZNF643 dans la cellule, et
b) mesurer la modulation, de préférence l'inhibition, de l'expression de Zfp69, ZNF642 et/ou ZNF643 dans ladite cellule,
dans lequel ledit composé est de préférence sélectionné à partir d'une bibliothèque de peptides, une bibliothèque combinatoire, un extrait cellulaire, en particulier un extrait cellulaire végétal, un « médicament à petites molécules », un oligonucléotide antisens, un ARNsi, un ARNm ou un acide nucléique codant pour *Zfp69, ZNF642* et/ou *ZNF643* ou une forme mutée de celui-ci, et un anticorps ou un fragment de celui-ci reconnaissant spécifiquement la séquence d'acides aminés de ZFP69, ZNF642 et/ou ZNF643.

10. Procédé conforme à la revendication 9, dans lequel ladite cellule est sélectionnée à partir du groupe consistant en des cellules du muscle squelettique, du foie, du tissu adipeux, du coeur, du pancréas, du rein et de l'hypothalamus.
